(19) Europäisches Patentamt
European Patent Office
Office européen des brevets

(11) EP 3 183 232 B1

(12) EUROPEAN PATENT SPECIFICATION

(45) Date of publication and mention of the grant of the patent:
**03.05.2023 Bulletin 2023/18**

(21) Application number: **15834369.9**

(22) Date of filing: **24.08.2015**

(51) International Patent Classification (IPC):
***C07D 211/58*** (2006.01) ***C07D 407/12*** (2006.01)
***C07D 409/06*** (2006.01) ***A61K 31/443*** (2006.01)
***A61K 31/4436*** (2006.01) ***A61K 31/44*** (2006.01)
***A61P 25/00*** (2006.01)

(52) Cooperative Patent Classification (CPC):
**C07D 211/58; A61K 31/16; A61K 31/4465; A61K 31/4468; A61K 31/451; A61P 25/00; C07D 405/12; C07D 407/12; C07D 409/06; C07D 409/12**

(86) International application number:
**PCT/US2015/046585**

(87) International publication number:
**WO 2016/029218 (25.02.2016 Gazette 2016/08)**

(54) **SUBSTITUTED 1-ARYLETHYL-4-ACYLAMINOPIPERIDINE DERIVATIVES AS OPIOID/ALPHA-ADRENORECEPTOR MODULATORS AND METHOD OF THEIR PREPARATION**

SUBSTITUIERTE 1-ARYLETHYL-4-ACYLAMINOPIPERIDIN-DERIVATE ALS OPIOID-/ALPHA-ADRENOREZEPTOR-MODULATOREN UND VERFAHREN ZU DEREN HERSTELLUNG

DÉRIVÉS DE 1-ARYLÉTHYL-4-ACYLAMINOPIPÉRIDINE SUBSTITUÉS À TITRE DE MODULATEURS DES RÉCEPTEURS D’OPIOÏDES/ADRÉNORÉCEPTEURS ALPHA ET LEUR PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.08.2014 US 201462040886 P**

(43) Date of publication of application:
**28.06.2017 Bulletin 2017/26**

(73) Proprietor: **The Arizona Board of Regents on behalf of the University of Arizona
Tucson, Arizona 85721-0300 (US)**

(72) Inventors:
- **VARDANYAN, Ruben S.
Tucson, Arizona 85721 (US)**
- **HRUBY, Victor J.
Tucson, Arizona 85721 (US)**

(74) Representative: **ZBM Patents - Zea, Barlocci & Markvardsen
Rambla Catalunya, 123
08008 Barcelona (ES)**

(56) References cited:
**WO-A1-2007/058482 US-A- 4 029 801
US-A- 4 029 801 US-A- 4 649 144
US-A1- 2004 147 503**

- **HARPER, N. J. ET AL.: "The Chemistry and Pharmacology of Some 4-Aminopiperidines and Their Derivatives", JOURNAL OF MEDICINAL CHEMISTRY, vol. 7, November 1964 (1964-11), pages 729-732, XP002779450, DOI: 10.1021/jm00336a010**
- **JOHN L. ARCHIBALD ET AL.: "Benzamidopiperidines. 3. Carbocyclic Derivatives Related to Indoramin", JOURNAL OF MEDICINAL CHEMISTRY, vol. 17, no. 7, 1974, pages 739-744, XP002779451, DOI: 10.1021/jm00253a017**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE PUBCHEM [Online] 25 January 2012 XP055409560 Retrieved from NCBI Database accession no. CID 56006844
- DATABASE PUBCHEM [Online] 23 October 2012 XP055409563 Retrieved from NCBI Database accession no. SID 150462038

## Description

The invention relates to novel pharmacological compounds, and more specifically to the creation of a new class of small molecules which simultaneously exhibit high binding affinities to the μ-, δ-, and κ-opioid receptors and the $\alpha_2$-adrenoreceptor. The binding activity is believed to be antagonistic at least with respect to the μ-opioid receptors. In addition to providing these compounds with novel pharmacological binding properties, the invention also describes detailed novel methods for the preparation of representative compounds and a scheme for the synthesis of related compounds that bind to the opioid receptors and/or $\alpha_2$-adrenoreceptor.

Opioid antagonists are drugs which bind to the opioid receptors with higher affinity than opioid agonists but do not activate the opioid receptors. Commonly known opioid antagonists include drugs such as, for example, naltrexone, naloxone, nelmefene, nalorphine, and nalbuphine. Opioid antagonists effectively block the receptor from the action of both naturally occurring agonists (e.g., morphine, codeine, thebaine) and synthetic agonists (e.g., fentanyl, pethidine, levorphanol, methadone, tramadol, dextropropoxyphene) and uses include counteracting life-threatening depression of the central nervous and respiratory systems and thus are used for emergency overdose and dependence treatment (e.g., naloxone). There are many excellent reviews dedicated to different aspects of opioid antagonists [28-46].

Opioid receptor antagonists are known to modulate numerous central and peripheral effects including those associated with opioid abuse, the development of opioid tolerance and dependence, opioid-induced constipation, alcohol and cocaine abuse, depression, and immune responses [1]. The diverse therapeutic applications of μ-opioid antagonists include opioid-overdose-induced respiratory depression, opioid and cocaine abuse, alcohol dependence, smoking cessation, obesity, psychosis[1-19] and for the treatment of dyskinesia associated with Parkinson's disease [20-27].

The few opioid antagonists currently on the market are represented by very few drugs (e.g., naloxone, naltrexone, and nalorphine (a partial agonist)) that have been shown to have therapeutic utility in a variety of indications. During last two decades only Alvimopan [13,14]-a peripherally acting μ-opioid antagonist for the treatment of postoperative ileus-has received approval as new drug. In addition, some azabicyclohexane derivatives and series of bi(hetero)aryl ethers as biological tools have been proposed as new chemical entities in this class of compounds [15].

Every chemical class of compounds with opioid-agonist activity has a structurally similar opioid-antagonist pair. Agonist-antagonist transformation in any of these cases takes place as a result of a small change in the structure of the agonist. The only exceptions, where the corresponding change for agonist-antagonist transformations has not been found, are the compounds of the fentanyl series.

Since the discovery of the "army" of opioid agonists of the fentanyl series (sufentanyl, alfentanyl, carfentanyl, remifentanyl, etc.) beginning in the 1960s, a structurally corresponding antagonist has not been found for any of these compounds. Thus, for decades there has been an evident gap in the art with respect to a possible specific structural change that could make possible the transformation of powerful opioid agonist properties of compounds of fentanyl series into powerful antagonists.

Similar to the general action of the opioid antagonists, antagonists of the adrenoreceptors (adrenergic receptors) bind to the adrenoreceptors and act to inhibit the action of those receptors. Alpha antagonists, or alpha-blockers, may selectively act at the $\alpha_1$-adrenoreceptors or at the $\alpha_2$-adrenoreceptors, or they may non-selectively act at both receptors. Commonly known α-blockers include, for example, phenoxybenzamine and phentolamine (non-selective); alfuzosin and prazosin ($\alpha_1$-blockers); and atipamezole, idazoxan, mirtazapine and yohimbine ($\alpha_2$-blockers). Generally, α-blockers have shown to be effective in the treatment of various medical conditions, including Raynaud's disease, hypertension, scleroderma, anxiety and panic disorders, and in the treatment of dyskinesia associated with Parkinson's disease.

The present invention is based on the discovery of certain compounds exhibiting high binding affinity for the μ-, δ-, and κ- opioid receptors and the $\alpha_2$-adrenoreceptor. The compounds are believed to exhibit antagonistic activity at least with respect to μ-opioid receptors. The compounds are structurally related to the fentanyl series of opioid receptor agonists. Processes for preparing these compounds are also included in this disclosure.

One aspect of the invention relates to a compound having the formula

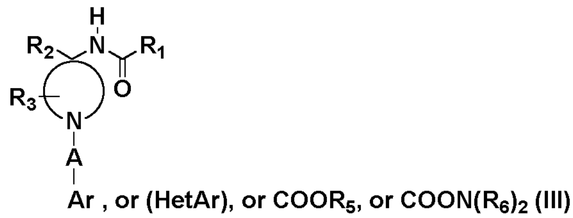

**(III)**

is disclosed, wherein $R_1$ is substituted or unsubstituted $C_1$-$C_{10}$ alkyl, alkenyl, or alkynyl, or substituted or unsubstituted aryl or hetaryl; $R_2$ is H, -$CH_2O$-$C_{1-4}$ alkyl; COO-$C_{1-4}$ alkyl; -$CONR_4$; $R_3$ is H, substituted or unsubstituted $C_1$-$C_{10}$ alkyl, alkynyl, or substituted or unsubstituted aryl or hetaryl; A is substituted or unsubstituted $C_1$-$C_{10}$ alkylene,

alkynylene; Ar or HetAr is substituted or unsubstituted monocyclic or polycyclic aromatic or heteroaromatic moiety; $COOR_5$, or $CON(R_6)_2$, where $R_5$ and $R_6$ are H, substituted or unsubstituted $C_1$-$C_{10}$ alkyl, alkynyl, or substituted or unsubstituted aryl or hetaryl; the central nitrogen-containing ring is a substituted or unsubstituted 5- to 7-membered heterocyclic ring; and pharmaceutically acceptable salts of said compound;
for use in the treatment of a clinical condition associated with opioid abuse, a development of opioid tolerance and dependence, opioid-induced constipation, alcohol abuse, opioid abuse, cocaine abuse, depression, opioid induced immune response depression, opioid-overdose-induced respiratory depression, nicotine withdrawal symptoms, obesity, psychosis, dyskinesia associated with Parkinson’s disease, Raynaud’s disease, scleroderma or a combination thereof, through modulation of $\mu$-, $\delta$-, k-opioid receptors and $\alpha$2-adrenoreceptors.

In a preferred embodiment, the prepared compound for use as defined above may belong to the series of N-(1-arylethylpiperidin-4-yl)acylamides. In another embodiment, the compound may be N-(1-phenethylpiperidin-4-yl)propionamide (Compound I, below). In yet another embodiment the compound may be the oxalate salt, or other pharmaceutically acceptable salt, of N-(1-phenethylpiperidin-4-yl)propionamide.

Another aspect of the invention relates to a compound having the formula (III) as defined above which is selected from the group consisting of:

*N*-(1-phenethylpiperidin-4-yl)propionamide;
*N*-(1-phenethylpiperidin-4-yl)propionamide oxalate;
*N*-(1-benzylpiperidin-4-yl)propionamide;
methyl 3-(4-propionamidopiperidin-1-yl)propanoate;
*N*-(1-phenethylpiperidin-4-yl)furan-2-carboxamide;
*N*-(1-phenethylpiperidin-4-yl)furan-3-carboxamide;
*N*-(1-(2-(thiophen-2-yl)ethyl)piperidin-4-yl)propionamide; and
8-bromo-N (1-phenethylpiperidin-4-yl)octanamide.

A process for preparing a compound of formula III is also disclosed. The process comprising the following steps: (a) reacting a cyclic ketone having a protecting group in a Grignard or Reformatsky reaction to obtain a first product; (b) reacting the product of step (a) in a Ritter reaction to obtain a second product; and (c) deprotecting the product of step (b) with acylation or alkylation to obtain a compound of formula III.

Another process for preparing a compound of formula III is also disclosed, comprising the following steps: (a) reacting a cyclic ketone having a protecting group in a Strecker reaction to obtain a first product; (b) reacting the product of step (a) in a selective carbalkoxy group transformation to obtain a second product; and (c) deprotecting the product of step (b) with acylation or alkylation to obtain a compound of formula III.

A process for preparing N-(1-phenethylpiperidin-4-yl) propionamide is also disclosed, comprising the following steps: (a) reacting phenethylpiperidin-4-one with hydroxylamine hydrochloride in ethanol in the presence of a base, to produce 1-phenethylpiperidin-4-one oxime; (b) reducing the oxime obtained in step (a) with iso-amyl alcohol and sodium metal to produce 1-phenethylpiperidin-4-amine; and (c) acylating the product of step (b) with propionic acid chloride in chloroform in the presence of triethylamine to produce N-(1-phenethylpiperidin-4-yl)propionamide. The N-(1-phenethylpiperidin-4-yl)propionamide may optionally be further treated with oxalic acid to obtain N-(1-phenethylpiperidin-4-yl)propionamide oxalate.

These and other embodiments, features and advantages of the present invention will become more fully apparent when read in conjunction with the following detailed description taken in conjunction with the accompanying drawings, wherein

Fig. 1 is a (LC/MS) plot of a preferred compound designated HCV-3.
Fig. 2 is a structural model of a unit cell of the Fig. 1 molecule, in which the oxalate molecule has been removed.
Fig. 3 is a structural model similar to Fig. 2, in which fog has been added to show depth.
Fig. 4 is a structural model of a unit cell, viewed down the short a axis.
Fig. 5 is a histogram plotting % of control agonist responses for HCV-3.
Fig. 6 is a histogram plotting inhibition of control agonist response for HCV-3.

For the purposes of this disclosure, a "salt" is any acid addition salt, preferably a pharmaceutically acceptable acid addition salt, including halogenic acid salts such as hydrobromic, hydrochloric, hydrofluoric and hydroiodic acid salt; an inorganic acid salt such as, for example, nitric, perchloric, sulfuric and phosphoric acid salt; an organic acid salt such as, for example, sulfonic acid salts (methanesulfonic, trifluoromethan sulfonic, ethanesulfonic, benzenesulfonic orp-toluenesulfonic), acetic, malic, fumaric, succinic, citric, benzoic, gluconic, lactic, mandelic, mucic, pamoic, pantothenic, oxalic and maleic acid salts; and an amino acid salt such as aspartic or glutamic acid salt. The acid addition salt may

be a mono- or di-acid addition salt, such as a di-hydrohalogenic, di-sulfuric, di-phosphoric or di-organic acid salt. In all cases, the acid addition salt is used as an achiral reagent which is not selected on the basis of any expected or known preference for interaction with or precipitation of a specific optical isomer of the products of this disclosure.

"Pharmaceutically acceptable salt" is meant to indicate those salts which are, within the scope of sound medical judgment, suitable for use in contact with the tissues of a patient without undue toxicity, irritation, allergic response, and are commensurate with a reasonable benefit/risk ratio. Pharmaceutically acceptable salts are well known in the art. For example, Berge et al. (1977) J. Pharm. Sciences, vol. 6. 1-19, which is hereby incorporated by reference in its entirety, describes pharmaceutically acceptable salts in detail.

"Modulation" is meant to refer to the binding activity of a compound with respect to a particular receptor. The binding activity of the compound, or "modulator," may be that of an agonist, inverse agonist, antagonist, allosteric regulator, positive allosteric modulator, negative allosteric modulator, or any other type of ligand-receptor interaction that is known in the art.

In this invention we disclose a new class of molecules that simultaneously bind with high affinity to opioid μ-, δ-, κ- receptors and also to α-adrenoreceptors, thereby exhibiting modulation-type interactions with those receptors. The interaction of the molecules with μ-receptors is believed to have the character of antagonist action, based at least in part on the observed high affinity binding of the molecules with respect to the μ-receptors.

Although not wishing to be bound by theory, it appears that the principal structural change for agonist-antagonist transformation is the removal of a phenyl group from an N-phenylpropionamide fragment of fentanyl. This transformation is depicted below, wherein N-(1-phenethylpiperidin-4-yl)-N-phenylpropionamide (II) is transformed to N-(1-phenethylpiperidin-4-yl)-N-propionamide (I), causing a transformation of μ-agonist properties to μ-antagonist with simultaneous modulation of delta-, kappa- and alpha-receptors:

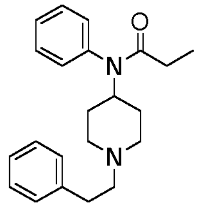

**Fentanil (II)**

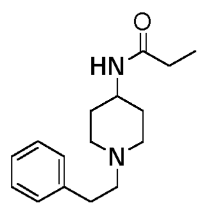

**μ-Opioid Antagonist/α-agonist (I)**

The invention also discloses processes for preparing compounds of general formula III, which are pharmacologically active compounds:

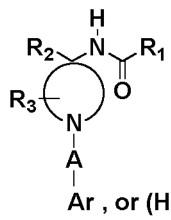

**Ar , or (HetAr), or COOR$_5$, or COON(R$_6$)$_2$ (III)**

Representative compounds of the present disclosure also include the following compounds, wherein the central nitrogen containing ring is a substituted or unsubstituted 5- to 7-membered heterocyclic ring:

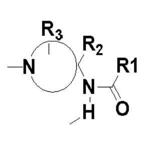 = 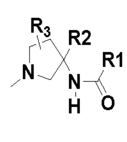 ; 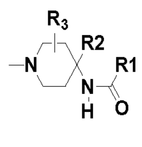 ; 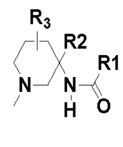 ; 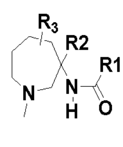 ; 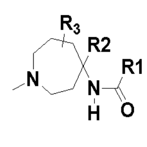

These compounds may include the following structural and functional groups:

- $R_1$ is H, substituted or unsubstituted $C_1$-$C_{10}$ alkyl, alkenyl, alkynyl, or substituted or unsubstituted aryl or hetaryl;
- $R_2$ is H, -$CH_2O$-$C_{1-4}$ alkyl; COO-$C_{1-4}$ alkyl; -$CONR_4$;
- $R_3$ is H, substituted or unsubstituted $C_1$-$C_{10}$ alkyl, alkynyl, or substituted or unsubstituted aryl or hetaryl;
- A is substituted or un-substituted $C_1$-$C_{10}$ alkylene, alkynylene;

- Ar or HetAr is substituted or un-substituted monocyclic or polycyclic aromatic or heteroaromatic moiety; and
- $COOR_5$, or $CON(R_6)_2$, where $R_5$ and $R_6$ are H, substituted or unsubstituted $C_1$-$C_{10}$ alkyl, alkynyl, or substituted or un-substituted aryl or hetaryl.

Compounds of formula (III) can be prepared according to the following general schemes (wherein PG is a protecting group):

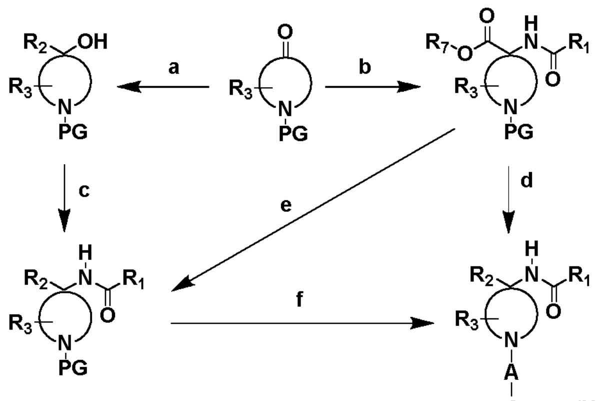


Ar , or (HetAr), or $COOR_5$, or $COON(R_6)_2$ (III)

As shown above, these schemes generally include transformations of different starting cyclic ketones (e.g., a variety of piperidin-4-ones, pyrrolidin-3-ones and azepan-4-one) to desired compounds of formula (III) via, for example:

a) Grignard or Reformatsky type reactions;
b) a Strecker type reaction;
c) a Ritter type reaction;
d) selective carbalkoxy group transformations, deprotection, and further appropriate acylation or alkylation;
e) selective carbalkoxy group transformations; or
f) deprotection with further appropriate acylation or alkylation.

The process for the preparation of the first example of μ-opioid modulator/α-modulator N-(1-phenethylpiperidin-4-yl)propionamide and its salt is described in the present invention in detail below (Scheme 1):

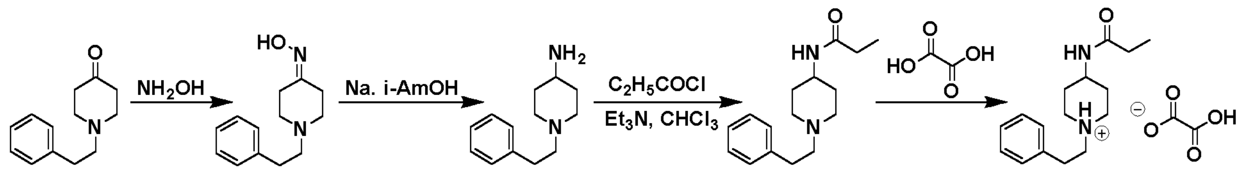


(IV) (V) (VI) (I) (VII)

The synthetic procedure starts with the commercially available 1-phenethylpiperidin-4-one (IV) of formula:

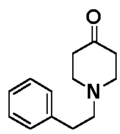 (IV)

which may be reacted with hydroxylamine hydrochloride in ethanol in the presence of base, to give 1-phenethylpiperidin-4-one oxime (V) of formula:

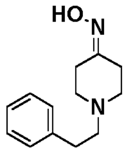

(V)

Reducing the C=N double bond of obtained oxime (V) by the Bouveault-Blanc protocol using iso-amyl alcohol and sodium metal as a hydrogen source, 1-phenethylpiperidin-4-amine (VI) may be obtained:

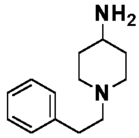

(VI)

By treating of compound (VI) with propionic acid chloride in chloroform in the presence of triethylamine the desired N-(1-phenethylpiperidin-4-yl)propionamide (I) may be prepared:

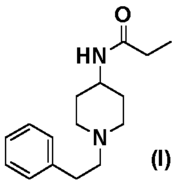

(I)

The obtained N-(1-phenethylpiperidin-4-yl)propionamide (I) may be transformed to oxalate by treating with a molar equivalent of oxalic acid in ethanol:

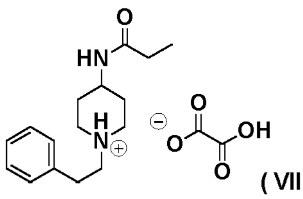

( VII )

(Tables 1-3) below include data from binding assays performed with the N-(1-phenethylpiperidin-4-yl)propionamide compound (I). This data demonstrates the high binding affinities of the compounds of the invention for μ-, δ-, and κ- opioid receptors and for α2-adreno-receptors. Table 1 illustrates the results of binding assays performed with the N-(1-phenethylpiperidin-4-yl)propionamide (compounds R1 and R2) and various receptors. As can be seen in the table, at a test concentration of 1.0E-05 M, N-(1-phenethylpiperidin-4-yl)propionamide demonstrated the highest percentage binding inhibition of control specific binding with respect to the α2B adrenoreceptor (74% and 55%); the δ-opioid receptor (44% and 69%); the κ-opioid receptor (107% and 104%); the μ-opioid receptor (98% and 99%). Table 2 contains reference compound data for the various receptors used in the binding assays. Finally, Table 3 provides summary results of the binding assays, showing the receptors from Table 1 for which the test compound demonstrated the highest percentage binding inhibition of control specific binding.

Study Number 8255

Table 1

Binding Assays

Summary Results

| Assay Cerep Compound I.D. | Client Compound I.D. | Test Concentration (M) | % Inhibition of Control Specific Binding |
|---|---|---|---|
| $\alpha_2$ (non-selective) | | | |

(continued)

| Binding Assays Summary Results Assay Cerep Compound I.D. | Client Compound I.D. | Test Concentration (M) | % Inhibition of Control Specific Binding |
|---|---|---|---|
| 8255-1 | RSA 101c | 1.0E-05 | 4 |
| 8255-2 | R1 | 1.0E-05 | 33 |
| 8255-3 | R2 | 1.0E-05 | 24 |
| $\alpha_{2B}$ | | | |
| 8255-1 | RSA 101c | 1.0E-05 | -21 |
| 8255-2 | R1 | 1.0E-05 | 74 |
| 8255-3 | R2 | 1.0E-05 | 55 |
| BZD (central) | | | |
| 8255-1 | RSA 101c | 1.0E-05 | 6 |
| 8255-2 | R1 | 1.0E-05 | 17 |
| 8255-3 | R2 | 1.0E-05 | 12 |
| CGRP (*h*) | | | |
| 8255-1 | RSA 101c | 1.0E-05 | -7 |
| 8255-2 | R1 | 1.0E-05 | -8 |
| 8255-3 | R2 | 1.0E-05 | 3 |
| $CB_1$ (h) | | | |
| 8255-1 | RSA 101c | 1.0E-05 | -3 |
| 8255-2 | R1 | 1.0E-05 | -3 |
| 8255-3 | R2 | 1.0E-05 | 12 |
| $CB_2$ (*h*) | | | |
| 8255-1 | RSA 101c | 1.0E-05 | 5 |
| 8255-2 | R1 | 1.0E-05 | 15 |
| 8255-3 | R2 | 1.0E-05 | 25 |
| $GABA_B$ | | | |
| 8255-1 | RSA 101c | 1.0E-05 | 6 |
| 8255-2 | R1 | 1.0E-05 | -1 |
| 8255-3 | R2 | 1.0E-05 | -11 |
| Galanin (non-selective) | | | |
| 8255-1 | RSA 101c | 1.0E-05 | -191 |
| 8255-2 | R1 | 1.0E-05 | -30 |
| 8255-3 | R2 | 1.0E-05 | -30 |
| $MC_1$ | | | |
| 8255-1 | RSA 101c | 1.0E-05 | 6 |
| 8255-2 | R1 | 1.0E-05 | 7 |
| 8255-3 | R2 | 1.0E-05 | 13 |
| $MC_4$ (*h*) | | | |
| 8255-1 | RSA 101c | 1.0E-05 | 46 |
| 8255-2 | R1 | 1.0E-05 | 1 |
| 8255-3 | R2 | 1.0E-05 | 2 |
| $NK_1$ (*h*) | | | |
| 8255-1 | RSA 101c | 1.0E-05 | -7 |
| 8255-2 | R1 | 1.0E-05 | 11 |
| 8255-3 | R2 | 1.0E-05 | 6 |
| $\delta$ (DOP) | | | |

(continued)

| Binding Assays Summary Results Assay Cerep Compound I.D. | Client Compound I.D. | Test Concentration (M) | % Inhibition of Control Specific Binding |
|---|---|---|---|
| 8255-1 | RSA 101c | 1.0E-05 | 98 |
| 8255-2 | R1 | 1.0E-05 | 44 |
| 8255-3 | R2 | 1.0E-05 | 69 |
| κ (KOP) | | | |
| 8255-1 | RSA 101c | 1.0E-5 | 79 |
| 8255-2 | R1 | 1.0E-5 | 107 |
| 8255-3 | R2 | 1.0E-5 | 104 |
| μ (*h*) (MOP) | | | |
| 8255-1 | RSA 101c | 1.0E-05 | 100 |
| 8255-2 | R1 | 1.0E-05 | 98 |
| 8255-3 | R2 | 1.0E-05 | 99 |
| ORL 1 (*h*) (NOP) | | | |
| 8255-1 | RSA 101c | 1.0E-05 | 4 |
| 8255-2 | R1 | 1.0E-05 | 22 |
| 8255-3 | R2 | 1.0E-05 | 32 |
| $TXA_2/PGH_2$ (*h*)(TP) | | | |
| 8255-1 | RSA 101C | 1.0E-05 | 13 |
| 8255-2 | R1 | 1.0E-05 | 14 |
| 8255-3 | R2 | 1.0E-05 | 18 |
| P2Y | | | |
| 8255-1 | RSA 101c | 1.0E-05 | -3 |
| 8255-2 | R1 | 1.0E-05 | -1 |
| 8255-3 | R2 | 1.0E-05 | -1 |
| σ (non-selective) | | | |
| 8255-1 | RSA 101c | 1.0E-05 | 33 |
| 8255-2 | R1 | 1.0E-05 | 104 |
| 8255-3 | R2 | 1.0E-05 | 101 |
| $K^+_v$ channel | | | |
| 8255-1 | RSA 101c | 1.0E-05 | -1 |
| 8255-2 | R1 | 1.0E-05 | -1 |
| 8255-3 | R2 | 1.0E-05 | 0 |
| NE transporter (h) | | | |
| 8255-1 | RSA 101c | 1.0E-05 | -7 |
| 8255-2 | R1 | 1.0E-05 | -16 |
| 8255-3 | R2 | 1.0E-05 | 29 |
| GABA transporter | | | |
| 8255-1 | RSA 101c | 1.0E-05 | 5 |
| 8255-2 | R1 | 1.0E-05 | -1 |
| 8255-3 | R2 | 1.0E-05 | 17 |

Study Number 8255

Table 2

Binding Assays

| Assay Reference Compound | $IC_{50}$ (M) | $K_i$ (M) | $n_u$ |
|---|---|---|---|
| $\alpha_2$ (non-selective) | | | |
| yohimbine | 4.7E-09 | 2.0E-08 | 1.1 |
| $\alpha_{2B}$ | | | |
| yohimbine | 9.1E-09 | 3.6E-09 | 1.3 |
| BZD (central) | | | |
| Diazepam | 2.1E-08 | 1.8E-09 | 1.1 |
| CGRP (*h*) | | | |
| hCGRP $\alpha$ | 4.0E-11 | 2.0E-11 | 1.0 |
| $CB_1$ (*h*) | | | |
| WIN 55212-2 | 2.4E-08 | 1.8E-08 | 1.2 |
| $CB_2$ (*h*) | | | |
| WIN 55212-2 | 2.9E-09 | 1.0E-09 | 0.9 |
| $GABA_B$ | | | |
| baclofen | 1.5E-07 | 8.1E-08 | 1.2 |
| Galanin (non-selective) | | | |
| galanin | 5.1E-10 | 3.4E-10 | 1.1 |
| galanin | 4.5E-10 | 3.0E-10 | 2.1 |
| $MC_1$ | | | |
| NDP- $\alpha$-MSH | 2.6E-10 | 1.3E-10 | 1.1 |
| $MC_4$ (*h*) | | | |
| NDP- $\alpha$-MSH | 3.5E-10 | 2.9E-10 | 1.0 |
| $NK_1$ (h) | | | |
| [$Sar^9$, $Met(O_2)^{11}$]-SP | 5.8E-10 | 2.6E 10 | 0.6 |
| $\delta$ (DOP) | | | |
| DPDPE | 3.2E-09 | 1.2E-09 | 1.0 |
| $\kappa$ (KOP) | | | |
| U 50488 | 1.5E-09 | 4.9E-10 | 2.0 |
| $\mu$ (h) (MOP) | | | |
| DAMGO | 2.1E-09 | 7.6E-10 | 0.7 |
| ORL 1 (*h*) (NOP) | | | |
| nociceptin | 7.6E-09 | 3.4E-09 | 1.5 |
| $TXA_2/PGH_2$ (*h*)(TP) | | | |
| U 44069 | 1.4E-07 | 9.0E-08 | 0.7 |
| U44069 | 3.9E-07 | 2.5E-07 | 0.6 |
| P2Y | | | |
| dATPa*S* | 2.4E-09 | 1.2E-08 | 0.8 |
| $\sigma$ (non-selective) | | | |
| haloperidol | 7.9E-08 | 6.2E-08 | 0.9 |
| $K^+_v$ channel | | | |
| $\alpha$-dendrotoxin | 7.7E-10 | 6.1E-10 | 2.8 |
| NE transporter (*h*) | | | |
| protriptyline | 1.0E-08 | 7.9E-09 | 1.1 |

Reference Compound Data

(continued)

Reference Compound Data

| Assay Reference Compound | $IC_{50}$ (M) | $K_i$ (M) | $n_u$ |
|---|---|---|---|
| GABA transporter | | | |
| nipecotic acid | 2.8E-06 | 2.7E-06 | 0.9 |

Study Number 8255

Table 3
Binding Assays
Summary Results

| Assay Cerep Compound I.D. | Client Compound I.D. | Test Concentration (M) | % Inhibition of Control Specific Binding |
|---|---|---|---|
| μ(*h*) (MOP) | | | |
| 8255-2 | R1 | 1.0E-05 | 98 |
| 8255-3 | R2 | 1.0E-05 | 99 |
| κ (KOP) | | | |
| 8255-2 | R1 | 1.0E-05 | 107 |
| 8255-3 | R2 | 1.0E-05 | 104 |
| σ (non-selective) | | | |
| 8255-2 | R1 | 1.0E-05 | 104 |
| 8255-3 | R2 | 1.0E-05 | 101 |
| $\alpha_{2B}$ | | | |
| 8255-2 | R1 | 1.0E-05 | 74 |
| 8255-3 | R2 | 1.0E-05 | 55 |

X-ray crystallography data for a representative compound of the invention, N-(1-phenethylpiperidin-4-yl)propionamide oxalate, is provided below:
The crystals submitted were small needles which diffracted poorly. Reflections were streaky, indicating that the crystal was not single but comprised of more than one not-exactly-oriented component. Diffraction was observed only to about 1 Å resolution. Because of the poor resolution, A and B level check if alerts are generated. The quality of the structure is such that the identity of the molecule and its conformation is confirmed, but derived parameters (bond distances, angles, thermal motion) are not reliable.

Nevertheless, the structure could be determined and refined, and the molecule is shown in Figure 2. Figure 3 shows the contents of the unit cell, which also includes an oxalate molecule.

Figure 4 shows the unit cell viewed down the crystallographic *a* axis. The hydrogen bonding network in the unit cell connects the organic molecule to oxalate along the crystallographic b axis (hydrogen bonds between the N1 and O2 of the oxalate and between N2 and O4 of the oxalate). The oxalate molecule are connected by hydrogen bonds O4 and O5 of the oxalate, along the crystallographic a axis.

C8 has been modeled with disorder in two positions. There is unmodeled (probably rotational) disorder in the aromatic ring (C11-C16). Both distance and planarity restraints were applied to this ring during refinement. Not all hydrogen atoms were visible in the electron density map. Because of the low resolution and streaky diffraction pattern, the structure could not be refined without constraints. Constraints used in the refinement are included supra.

**References:**

**APEX2** (data collection)
Bruker (2007). APEX2. Bruker AXS Inc., Madison, Wisconsin, USA.
**SAINT** (integration and reduction)

Bruker (2007). SAINT. Bruker AXS Inc., Madison, Wisconsin, USA.
**SADABS** (absorption correction)
Sheldrick, G. M. (1996). SADABS. University of Göttingen, Germany.
**SHELXTL** (structure solution and refinement)
Sheldrick, G. M. (2008). Acta Cryst. A64, 112-122.
**MERCURY** (molecular graphics - hydrogen bonding and packing)
Macrae, C. F., Bruno, I. J., Chisholm, J. A., Edgington, P. R., McCabe, P. Pidcock, E., Rodriguez-Monge, L. Taylor, R., van de Streek, J. & Wood, P. A. (2008). J. Appl. Cryst. 41, 466-470.
**PLATON**
Spek, A. L. (2003). J. Appl. Cryst. 36, 7-13.
**OLEX2**
Dolomanov, O.V., Bourhis, L.J., Gildea, R.J., Howard, J.A.K., Puschmann H. (2008), J. Appl. Cryst. 42, 339-341.

**Table 4** Crystal data and structure refinement

| | |
|---|---|
| Identification code | rv101 |
| Empirical formula | $C_{18}H_{27}N_2O_5$ |
| Formula weight | 351.41 |
| Temperature/K | 150.0 |
| Crystal system | triclinic |
| Space group | P-1 |
| a/Å | 5.701(3) |
| b/Å | 12.024(6) |
| c/Å | 14.105(7) |
| $\alpha$/° | 100.473(14) |
| $\beta$/° | 93.997(14) |
| $\gamma$/° | 95.571(15) |
| Volume/Å$^3$ | 942.5(8) |
| Z | 2 |
| $\rho_{calc}$g/cm$^3$ | 1.238 |
| $\mu$/mm$^{-1}$ | 0.090 |
| F(000) | 378.0 |
| Crystal size/mm$^3$ | 0.3 × 0.08 × 0.05 |
| Radiation | MoK$\alpha$ ($\lambda$ = 0.71073) |
| 2$\Theta$ range for data collection/° | 2.948 to 41.622 |
| Index ranges | $-5 \leq h \leq 5$, $-11 \leq k \leq 11$, $-14 \leq 1 \leq 14$ |
| Reflections collected | 7408 |
| Independent reflections | 1965 [$R_{int}$ = 0.0625, $R_{sigma}$ = 0.0684] |
| Data/restraints/parameters | 1965/237/222 |
| Goodness-of fit on F$^2$ | 1.592 |
| Final R indexes [I>=2$\sigma$ (I)] | $R_1$ = 0.0970, w$R_2$ = 0.2383 |
| Final R indexes [all data] | $R_1$ = 0.1319, w$R_2$ = 0.2545 |
| Largest diff. peak/hole / e Å$^{-3}$ | 0.57/-0.46 |

**Table 5** Fractional Atomic Coordinates (×10$^4$) and Equivalent Isotropic Displacement Parameters (Å×10$^3$) $U_{eq}$ is defined as 1/3 of the trace of the orthogonalised $U_{IJ}$ tensor.

| **Atom** | ***x*** | ***y*** | ***z*** | **U(eq)** |
|---|---|---|---|---|
| O4 | 2499(7) | 898(4) | 1556(3) | 26.4(12) |
| O5 | -3506(7) | 78(4) | 1467(3) | 28.1(12) |
| O3 | -1621(7) | 1811(4) | 1840(3) | 34.3(13) |
| O2 | 605(7) | -782(4) | 1662(3) | 29.6(13) |
| N1 | -2989(8) | -2451(4) | 1622(3) | 20.6(13) |
| N2 | -2795(9) | -3369(4) | -1448(4) | 25.0(14) |

(continued)

$U_{eq}$ is defined as 1/3 of the trace of the orthogonalised $U_{IJ}$ tensor.

| Atom | *x* | *y* | *z* | U(eq) |
|---|---|---|---|---|
| C2 | -1073(11) | -3368(6) | 206(5) | 26.8(16) |
| O1 | -2673(8) | -5260(4) | -1872(3) | 35.3(13) |
| C1 | -1627(11) | -3398(5) | 1239(5) | 27.3(17) |
| C3 | -3356(11) | -3435(5) | -460(5) | 26.4(16) |
| C9 | -3409(12) | -2486(6) | 2654(4) | 28.0(17) |
| C4 | -4744(11) | -2458(6) | -50(5) | 25.9(16) |
| C18 | -1623(11) | 803(6) | 1645(4) | 16.6(14) |
| C5 | -5248(10) | -2510(6) | 986(4) | 22.8(16) |
| C17 | 690(11) | 242(6) | 1604(4) | 16.3(14) |
| C7 | -1722(13) | -4117(6) | -3046(5) | 38.1(19) |
| C10 | -4412(13) | -1434(6) | 3188(5) | 36.6(19) |
| C6 | -2419(11) | -4299(6) | -2080(5) | 24.0(16) |
| C11 | -4740(9) | -1569(5) | 4222(3) | 39.1(18) |
| C12 | -2894(8) | -1194(5) | 4941(4) | 62(3) |
| C13 | -3168(9) | -1341(5) | 5883(3) | 76(3) |
| C14 | -5288(10) | -1864(5) | 6107(3) | 59(2) |
| C15 | -7134(8) | -2239(6) | 5388(4) | 120(5) |
| C16 | -6860(8) | -2091(6) | 4445(4) | 114(5) |
| C8 | -1320(80) | -5210(40) | -3670(30) | 66(3) |
| C8A | 680(20) | -4565(10) | -3240(8) | 66(3) |

**Table** 6 Anisotropic Displacement Parameters ($Å^2 \times 10^3$).
The Anisotropic displacement factor exponent takes the form:
$-2\pi^2[h^2a^{*2}U_{11}+2hka^*b^*U_{12}+...]$.

| Atom | $U_{11}$ | $U_{22}$ | $U_{33}$ | $U_{23}$ | $U_{13}$ | $U_{12}$ |
|---|---|---|---|---|---|---|
| O4 | 7(2) | 29(3) | 48(3) | 18(2) | 4(2) | 7.9(19) |
| O5 | 10(2) | 29(3) | 47(3) | 13(2) | 4(2) | 2.6(19) |
| O3 | 20(3) | 26(3) | 57(3) | 6(2) | 6(2) | 4(2) |
| O2 | 14(2) | 23(2) | 57(3) | 15(2) | 9(2) | 7.7(19) |
| N1 | 9(3) | 21(3) | 34(3) | 11(2) | -1(2) | 5(2) |
| N2 | 18(3) | 24(3) | 36(3) | 11(2) | 7(2) | 5(2) |
| C2 | 17(3) | 24(4) | 43(4) | 12(3) | 5(3) | 11(3) |
| O1 | 37(3) | 23(3) | 48(3) | 9(2) | 5(2) | 6(2) |
| C1 | 12(3) | 26(4) | 43(4) | 6(3) | -1(3) | 4(3) |
| C3 | 16(3) | 27(4) | 40(3) | 13(3) | 4(3) | 4(3) |
| C9 | 17(4) | 36(4) | 33(3) | 15(3) | 0(3) | 3(3) |
| C4 | 17(4) | 29(4) | 36(3) | 11(3) | 7(3) | 7(3) |
| C18 | 9(2) | 20(3) | 22(3) | 7(2) | 3(2) | 2(2) |
| C5 | 9(3) | 26(4) | 36(3) | 13(3) | -1(3) | 6(3) |
| C17 | 7(2) | 23(3) | 22(3) | 9(2) | 5(2) | 4(2) |
| C7 | 47(4) | 39(4) | 35(4) | 14(3) | 7(3) | 15(4) |
| C10 | 37(4) | 41(4) | 38(4) | 14(3) | 6(3) | 18(4) |
| C6 | 13(4) | 26(3) | 36(3) | 9(3) | 0(3) | 8(3) |
| C11 | 32(4) | 49(5) | 43(4) | 16(3) | 9(3) | 20(3) |
| C12 | 60(5) | 81(7) | 43(4) | 17(4) | -1(3) | -12(5) |
| C13 | 88(6) | 98(7) | 39(4) | 13(4) | 4(4) | 2(5) |
| C14 | 66(5) | 72(6) | 50(4) | 20(4) | 19(3) | 42(4) |
| C15 | 55(5) | 238(14) | 77(5) | 77(6) | 11(4) | -20(7) |

(continued)

The Anisotropic displacement factor exponent takes the form: $-2\pi^2[h^2a^{*2}U_{11}+2hka^*b^*U_{12}+...]$.

| Atom | $U_{11}$ | $U_{22}$ | $U_{33}$ | $U_{23}$ | $U_{13}$ | $U_{12}$ |
|---|---|---|---|---|---|---|
| C16 | 40(5) | 238(14) | 72(5) | 78(7) | -6(4) | -29(6) |
| C8 | 72(7) | 78(7) | 59(6) | 17(5) | 26(5) | 35(6) |
| C8A | 72(7) | 78(7) | 59(6) | 17(5) | 26(5) | 35(6) |

**Table 7** Bond Lengths

| Atom | Atom | Length/Å | Atom | Atom | Length/Å |
|---|---|---|---|---|---|
| O4 | C17 | 1.250(7) | C9 | C10 | 1.532(9) |
| O5 | C18 | 1.295(7) | C4 | C5 | 1.519(8) |
| O3 | C18 | 1.194(7) | C18 | C17 | 1.538(9) |
| O2 | C17 | 1.245(7) | C7 | C6 | 1.493(9) |
| N1 | C1 | 1.482(7) | C7 | C8 | 1.49(4) |
| N1 | C9 | 1.499(8) | C7 | C8A | 1.542(13) |
| N1 | C5 | 1.505(7) | C10 | C11 | 1.519(7) |
| N2 | C3 | 1.465(8) | C11 | C12 | 1.3900 |
| N2 | C6 | 1.342(8) | C11 | C16 | 1.3900 |
| C2 | C1 | 1.519(9) | C12 | C13 | 1.3900 |
| C2 | C3 | 1.538(9) | C13 | C14 | 1.3900 |
| O1 | C6 | 1.242(7) | C14 | C15 | 1.3900 |
| C3 | C4 | 1.529(8) | C15 | C16 | 1.3900 |

**Table 8** Bond Angles

| Atom | Atom | Atom | Angle/° | Atom | Atom | Atom | Angle/° |
|---|---|---|---|---|---|---|---|
| C1 | N1 | C9 | 109.4(5) | O2 | C17 | O4 | 126.8(6) |
| C1 | N1 | C5 | 110.0(5) | O2 | C17 | C18 | 118.2(6) |
| C9 | N1 | C5 | 112.8(5) | C6 | C7 | C8A | 110.5(6) |
| C6 | N2 | C3 | 121.5(6) | C8 | C7 | C6 | 111.4(17) |
| C1 | C2 | C3 | 111.0(5) | C11 | C10 | C9 | 109.3(5) |
| N1 | C1 | C2 | 111.3(5) | N2 | C6 | C7 | 116.7(6) |
| N2 | C3 | C2 | 110.4(5) | O1 | C6 | N2 | 121.3(6) |
| N2 | C3 | C4 | 110.9(5) | O1 | C6 | C7 | 122.0(6) |
| C4 | C3 | C2 | 108.4(5) | C12 | C11 | C10 | 119.9(4) |
| N1 | C9 | C10 | 114.4(5) | C12 | C11 | C16 | 120.0 |
| C5 | C4 | C3 | 110.5(5) | C16 | C11 | C10 | 120.1(4) |
| O5 | C18 | C17 | 113.5(5) | C13 | C12 | C11 | 120.0 |
| O3 | C18 | O5 | 124.7(6) | C14 | C13 | C12 | 120.0 |
| O3 | C18 | C17 | 121.7(6) | C13 | C14 | C15 | 120.0 |
| N1 | C5 | C4 | 111.1(5) | C16 | C15 | C14 | 120.0 |
| O4 | C17 | C18 | 114.9(6) | C15 | C16 | C11 | 120.0 |

**Table 9** Torsion Angles

| A | B | C | D | Angle/° | A | B | C | D | Angle/° |
|---|---|---|---|---|---|---|---|---|---|
| O5 | C18 | C17 | O4 | 164.8(5) | C9 | C10 | C11 | C16 | -89.5(6) |
| O5 | C18 | C17 | O2 | -18.1(8) | C5 | N1 | C1 | C2 | 57.9(7) |
| O3 | C18 | C17 | O4 | -16.5(8) | C5 | N1 | C9 | C10 | -67.5(7) |

(continued)

| A | B | C | D | Angle/° | A | B | C | D | Angle/° |
|---|---|---|---|---|---|---|---|---|---|
| O3 | C18 | C17 | O2 | 160.6(6) | C10 | C11 | C12 | C13 | -178.4(5) |
| N1 | C9 | C10 | C11 | -179.6(5) | C10 | C11 | C16 | C15 | 178.4(5) |
| N2 | C3 | C4 | C5 | -178.1(5) | C6 | N2 | C3 | C2 | 85.2(7) |
| C2 | C3 | C4 | C5 | -56.7(7) | C6 | N2 | C3 | C4 | -154.6(6) |
| C1 | N1 | C9 | C10 | 169.7(5) | C11 | C12 | C13 | C14 | 0.0 |
| C1 | N1 | C5 | C4 | -58.3(6) | C12 | C11 | C16 | C15 | 0.0 |
| C1 | C2 | C3 | N2 | 178.2(5) | C12 | C13 | C14 | C15 | 0.0 |
| C1 | C2 | C3 | C4 | 56.5(7) | C13 | C14 | C15 | C16 | 0.0 |
| C3 | N2 | C6 | O1 | 4.4(9) | C14 | C15 | C16 | C11 | 0.0 |
| C3 | N2 | C6 | C7 | -176.5(6) | C16 | C11 | C12 | C13 | 0.0 |
| C3 | C2 | C1 | N1 | -58.2(7) | C8 | C7 | C6 | N2 | 179(2) |
| C3 | C4 | C5 | N1 | 58.5(7) | C8 | C7 | C6 | O1 | -2(2) |
| C9 | N1 | C1 | C2 | -177.7(5) | C8A | C7 | C6 | N2 | 122.0(8) |
| C9 | N1 | C5 | C4 | 179.3(5) | C8A | C7 | C6 | O1 | -58.9(10) |
| C9 | C10 | C11 | C12 | 88.9(6) | | | | | |

**Table 10** Hydrogen Atom Coordinates (Å×$10^4$) and Isotropic Displacement Parameters (Å$^2$×$10^3$)

| **Atom** | ***x*** | ***y*** | ***z*** | **U(eq)** |
|---|---|---|---|---|
| H4 | 3698 | 545 | 1578 | 40 |
| H5 | -3360 | -442 | 1785 | 42 |
| H1 | -2014 | -1718 | 1607 | 25 |
| H2 | -2702 | -2706 | -1630 | 30 |
| H2A | -188 | -4016 | -34 | 32 |
| H2B | -59 | -2656 | 191 | 32 |
| H1A | -2553 | -4133 | 1259 | 33 |
| H1B | -131 | -3342 | 1655 | 33 |
| H3 | -4332 | -4175 | -469 | 32 |
| H9A | -1893 | -2570 | 3007 | 34 |
| H9B | -4520 | -3167 | 2668 | 34 |
| H4A | -3821 | -1722 | -67 | 31 |
| H4B | -6255 | -2508 | -454 | 31 |
| H5A | -6132 | -1868 | 1242 | 27 |
| H5B | -6247 | -3228 | 997 | 27 |
| H7AA | -2983 | -3771 | -3369 | 46 |
| H7AB | -255 | -3582 | -2958 | 46 |
| H7BC | -1605 | -3295 | -3067 | 46 |
| H7BD | -2948 | -4520 | -3558 | 46 |
| H10A | -3313 | -744 | 3188 | 44 |
| H10B | -5951 | -1347 | 2854 | 44 |
| H12 | -1446 | -837 | 4788 | 75 |
| H13 | -1907 | -1085 | 6375 | 91 |
| H14 | -5475 | -1965 | 6751 | 71 |
| H15 | -8583 | -2596 | 5540 | 144 |
| H16 | -8121 | -2347 | 3954 | 137 |
| H8A | -734 | -5723 | -3269 | 100 |
| H8B | -150 | -5058 | -4125 | 100 |
| H8C | -2812 | -5564 | -4039 | 100 |
| H8AA | 1887 | -4172 | -2730 | 100 |
| H8AB | 1128 | -4424 | -3869 | 100 |

(continued)

| Atom | x | y | z | U(eq) |
|---|---|---|---|---|
| H8AC | 543 | -5385 | -3244 | 100 |

**Table 11** Atomic Occupancy

| Atom | *Occupancy* | Atom | *Occupancy* | Atom | *Occupancy* |
|---|---|---|---|---|---|
| H7AA | 0.215(12) | H7AB | 0.215(12) | H7BC | 0.785(12) |
| H7BD | 0.785(12) | C8 | 0.215(12) | H8A | 0.215(12) |
| H8B | 0.215(12) | H8C | 0.215(12) | C8A | 0.785(12) |
| H8AA | 0.785(12) | H8AB | 0.785(12) | H8AC | 0.785(12) |

**Experimental Details**

Needle shaped crystals of $C_{18}H_{27}N_2O_5$ were submitted for structure determination. The crystals were small and were grown together. Even apparently single crystals were stacks of needles A crystal was selected and attached to a Micromount using paratone oil, then mounted on a Bruker Kappa APEX-II Duo diffractometer. The crystal was kept at 150.0 K during data collection. Using Olex2 [1], the structure was solved with the ShelXS [2] structure solution program using Direct Methods and refined with the ShelXL [3] refinement package using Least Squares minimisation.

1. Dolomanov, O.V., Bourhis, L.J., Gildea, R.J, Howard, J.A.K. & Puschmann, H. (2009), J. Appl. Cryst. 42, 339-341.
2. Sheldrick, G.M. (2008). Acta Cryst. A64, 112-122.
3. Sheldrick, G.M. (2008). Acta Cryst. A64, 112-122.

**Crystal Data** for $C_{18}H_{27}N_2O_5$ ($M$=351.41 g/mol): triclinic, space group P-1 (no. 2), $a$ = 5.701(3) Å, $b$ = 12.024(6) Å, $c$ = 14.105(7) Å, $\alpha$ = 100.473(14)°, $\beta$ = 93.997(14)°, $\gamma$ = 95.571(15)°, $V$ = 942.5(8) Å$^3$, $Z$ = 2, $T$ = 150.0 K, $\mu$(MoK$\alpha$) = 0.090 mm$^{-1}$, $Dcalc$ = 1.238 g/cm$^3$, 7408 reflections measured (2.948° $\leq 2\Theta \leq$ 41.622°), 1965 unique ($R_{int}$ = 0.0625, $R_{sigma}$ = 0.0684) which were used in all calculations. The final $R_1$ was 0.0970 (I > 2$\sigma$(I)) and $wR_2$ was 0.2545 (all data).

**Refinement model description**

Number of restraints - 237, number of constraints -

Details:

1. Fixed Uiso
At 1.2 times of:
All C(H) groups, All C(H,H) groups, All C(H,H,H,H) groups, All N(H) groups
At 1.5 times of:
All C(H,H,H) groups, All O(H) groups
2. Restrained planarity
C11, C12, C13, C14, C15, C16, C10
with sigma of 0.1
3. Rigid bond restraints
O2, O5, C18, O4, C17, O3
with sigma for 1-2 distances of 0.01 and sigma for 1-3 distances of 0.01 C11, C16, C15, C14, C13, C12
with sigma for 1-2 distances of 0.01 and sigma for 1-3 distances of 0.01
4. Uiso/Uaniso restraints and constraints
Uanis(O3) ≈ Ueq, Uanis(C18) ≈ Ueq, Uanis(O5) ≈ Ueq, Uanis(O2) ≈ Ueq, Uanis(C17) ≈ Ueq, Uanis(O4) ≈ Ueq: with sigma of 0.005 and sigma for terminal atoms of 0.01

$$\mathrm{Uanis(C8)} = \mathrm{Uanis(C8A)}$$

5. Rigid body (RIGU) restrains
All non-hydrogen atoms
with sigma for 1-2 distances of 0.004 and sigma for 1-3 distances of 0.004
6. Others

Sof(H7BC)=Sof(H7BD)=Sof(C8A)=Sof(H8AA)=Sof(H8AB)=Sof(H8AC)=1-FVAR(1)

Sof(H7AA)=Sof(H7AB)=Sof(C8)=Sof(H8A)=Sof(H8B)=Sof(H8C)=FVAR(1)

7.a Ternary CH refined with riding coordinates:
N1(H1), C3(H3)
7.b Secondary CH2 refined with riding coordinates:
C2(H2A,H2B), C1(H1A,H1B), C9(H9A,H9B), C4(H4A,H4B), C5(H5A,H5B), C7(H7AA, H7AB), C7(H7BC,H7BD), C10(H10A,H10B)
7.c Aromatic/amide H refined with riding coordinates:
N2(H2), C12(H12), C13(H13), C14(H14), C15(H15), C16(H16)
7.d Fitted hexagon refined as free rotating group:
C11(C12,C13,C14,C15,C16)
7.e Idealised Me refined as rotating group:
C8(H8A,H8B,H8C), C8A(H8AA,H8AB,H8AC)
7.f Idealised tetrahedral OH refined as rotating group:
O4(H4), O5(H5)

Following is data showing that a representative compound of the invention, N-(1-phenethylpiperidin-4-yl)propionamide, conforms to "Lipinski's rule of five," which provides a general rule of thumb for evaluating the activity of an orally administered drug.

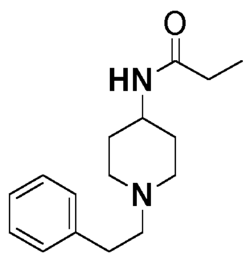

**μ-Opioid Antagonist/α-agonist (I)**

| Lipinski and Related Properties | Value | Condition | Note |
|---|---|---|---|
| Freely Rotatable Bonds | 5 | | (1) |
| H Acceptors | 3 | | (1) |
| H Donors | 1 | | (1) |
| H Donor/Acceptor Sum | 4 | | (1) |
| logP | 2.216±0.355 | Temp: 25 °C | (1) |
| Molecular Weight | 260.37 | | (1) |

**Lipinski's rule of five**

**Lipinski's rule of five** also known as the **Pfizer's rule of five** or simply the **Rule of five** (ROS) is a rule of thumb to evaluate druglikeness or determine if a chemical compound with a certain pharmacological or biological activity has properties that would make it a likely orally active drug in humans. The rule was formulated by Christopher A. Lipinski in 1997, based on the observation that most orally administered drugs are relatively small and moderately lipophilic molecules.

The rule describes molecular properties important for a drug's pharmacokinetics in the human body, including their absorption, distribution, metabolism,
and excretion ("ADME"). However, the rule does not predict if a compound is pharmacologically active.

The rule is important to keep in mind during drug discovery when a pharmacologically active lead structure is optimized step-wise to increase the activity and selectivity of the compound as well as to ensure drug-like physicochemical properties are maintained as described by Lipinski's rule.[3] Candidate drugs that conform to the RO5 tend to have lower attrition rates during clinical trials and hence have an increased chance of reaching the market.

Components of the rule]

Lipinski's rule states that, in general, an orally active drug has no more than one violation of the following criteria:

- No more than 5 hydrogen bond donors (the total number of nitrogen-hydrogen and oxygen-hydrogen bonds)
- Not more than 10 hydrogen bond acceptors (all nitrogen or oxygen atoms)
- A molecular mass less than 500 daltons
- An octanol-water partition coefficient[5] log *P* not greater than 5

Note that all numbers are multiples of five, which is the origin of the rule's name. As with many other rules of thumb, (such as Baldwin's rules for ring closure), there are many exceptions to Lipinski's Rule.

The compounds of the present invention may be utilized in various pharmaceutical and medical applications in which the use of a compound exhibiting high binding affinities for μ-, δ- or κ- opioid receptors and/or α2-adreno-receptors is indicated. The compounds may be of particular use in applications in which the use of a μ-, δ- or κ- opioid receptor modulator, including particularly a μ-opioid receptor antagonist, and/or an α2-adrenoreceptor modulator is indicated. Accordingly, in certain embodiments, the present invention also includes salts, and particularly pharmaceutically acceptable salts, of the disclosed compounds, as well as processes for preparing the salt forms of the disclosed compounds.

Examples

The following examples illustrate the preparation of N-(1-phenethylpiperidin-4-yl)propionamide and its oxalate salt form, N-(1-phenethylpiperidin-4-yl)propionamide oxalate, in accordance with the present disclosure.

**Example 1.**

1-Phenethylpiperidin-4-one oxime (Compound V)

1-Phenethylpiperidin-4-one (10.15 g (0.05 mol) dissolved in 60 mL of ethanol) was added drop-wise at 0°C to a solution of hydroxylamine in water. The water solution of hydroxylamine was preliminarily prepared by adding at 0°C in portions 13.8 g (0.1 mol) of $K_2CO_3$ to the solution of 6.95 g (0.1 mol) hydroxylamine hydrochloride in 50 mL of water.

The mixture was set aside for a night. Ethanol was evaporated under slight vacuum. Water (~100 mL) was added, and the mixture was stirred on ice bath for an hour. The separated solid product was filtered, washed with water and allowed to air-dry. The crude oxime (10.71 g (98.25%), m.p. 132-134°C) was reserved for use in the next reaction without further purification. Analysis with electrospray ionization mass spectrometry (MS (ESI)) resulted in a peak at 219.1 (MH+).

**Example 2**

1-Phenethylpiperidin-4-amine (Compound VI)

1-Phenethylpiperidin-4-one oxime (6.54 g (0.03 mol)) was dissolved in 100 mL of dry i-AmOH on heating. A ten-fold excess of sodium (6.9 g (0.3 mol)) was slowly (1 hour) added to the stirred solution in small pieces, while the

temperature was maintained around 110°. The solution was stirred on heating at 110° for two hours and left to cool to room temperature. 150 mL of ether, followed by 75 mL of water, was then added to the solution. The organic layer was separated and dried on $MgSO_4$. After evaporation of solvents under slight vacuum, the product was distilled to give 4.3 g (70%) of 1-phenethylpiperidin-4-amine (VI) with a boiling point of 138-142°/1.5mm. MS (ESI): 205.0 (MH+).

**Example 3**

N-(1-Phenethylpiperidin-4-yl)propionamide (Compound I)

Propionyl chloride (2.775 g (0.03 mol)) in 5.55 mL of $CHCl_3$ was added dropwise on stirring to the cooled (0°C) solution of 4.08 g (0.02 mol) 1-phenethylpiperidin-4-amine and 3.03 g (0.03 mol) of $Et_3N$ in 30 mL of $CHCl_3$. The mixture was left to come to room temperature and stirred overnight. After working up with 5% solution of $NaHCO_3$ (2.52 g (0.03 mol)) in 47.88 $H_2O$, the organic layer was separated, washed with water and dried on $MgSO_4$. After evaporation of solvents under slight vacuum, the residue was crystallized from hexane to give 4.9 g (94%) of N-(1-phenethylpiperidin-4-yl)propionamide (I) with m.p.134-135°. MS (ESI): 261.2 (MH+).

- The results of proton NMR spectroscopy were as follows:
  $^1$H NMR (600 MHz, $CDCl_3$): $\delta$ 7.27 (t, J = 7.4 Hz, 2H), 7.19 (m, 3H), 5.32 (d, J = 7.4 Hz, 1H), 3.82 (qt, J = 7.8, 4.2 Hz, 1H), 2.92 (dt, J = 11.8, 3.4 Hz, 2H), 2.79 (m*, 2H), 2.59 (m*, 2H), 2.19 (q, J = 7.5 Hz, 2H), 2.18 (m, 2H), 1.95 (dtd, J = 12.4, 4.4, 1.7 Hz, 2H), 1.46 (qd, J = 11.7, 3.8 Hz, 2H), 1.15 (t, J = 7.5 Hz, 3H).

  * these two multiplets arise from two methylene groups that have magnetically inequivalent protons AA'BB' with $^2J_{AA'}$ = $^2J_{BB'}$ = 12 Hz, $^3J_{AB}$ = $3J_{A'B'}$ = 11 Hz, $^3J_{AB'}$ = $^3J_{A'B}$ = 4 Hz, consistent with a preferred *anti* conformation for the phenyl and piperidine rings

- The results of carbon-13 NMR spectroscopy were as follows:
  $^{13}$C NMR (150 MHz, $CDCl_3$): $\delta$ 173.0, 140.2, 128.6, 128.4, 126.0, 60.4, 52.3, 46.3, 33.7, 32.3, 29.8, 9.9.

**Example 4**

N-(1-Phenethylpiperidin-4-yl)propionamide oxalate (Compound VII)

Oxalic acid (1 g (0.011 mol)) in 10 mL of ethanol was added drop-wise to the solution of 2.93 g (0.011 mol) of N-(1-phenethylpiperidin-4-yl)propionamide (I) in 29.3 mL of ethanol. The mixture was set aside overnight. The obtained crystals were then separated and dried in a desiccator over $P_2O_5$ to give 3.5 g of N-(1-phenethylpiperidin-4-yl)propionamide oxalate (VII) with m.p. 216-218 (MS (ESI): 261.2 ([M + H]) - see Fig. 1

The compound designated as HCV-3 was then subject to cellular functional assay and results reported below:

Cellular functional assays

| Experimental Assay | Catalog Ref Client Com Batch | | Compound Test Conce % of Contro% of Agonist Response | | |
|---|---|---|---|---|---|
| 27/07/201 alpha 2B (h1813 | HCV-3 | 1 | 1000233221.0E-05 | 7.1 | |
| 27/07/201 kappa (KO 2071 | HCV-3 | 1 | 1000233221.0E-05 | -4.2 | |
| 27/07/201 mu (MOP) | 1392 | HCV-3 | 1 | 1000233221.0E-05 | 33.5 |

| 1st | 2nd | Mean | Reference EC50 Ref(M) | |
|---|---|---|---|---|
| 9.2 | 5.1 | 7.1 | dexmedetc | 1.3E-08 |
| -10.3 | 1.8 | -4.2 | U 50488 | 1.6E-09 |
| 28.1 | 38.9 | 33.5 | DAMGO | 4.2E-09 |

Cellular functional assays

| Experimental Assay | Catalog Ref Client Com Batch | | Compound Test Conce % Inhibition Agonist Response (%of Control) | |
|---|---|---|---|---|
| 27/07/201 alpha 2B (h1814 | HCV-3 | 1 | 1000233221.0E-05 | -28 |
| 27/07/201 kappa (KO 2072 | HCV-3 | 1 | 1000233221.0E-05 | 6 |
| 27/07/201 mu (MOP) 1393 | HCV-3 | 1 | 1000233221.0E-05 | -1 |

| 1st | 2nd | Mean | Reference IC50Ref(NKbRef(M) | | |
|---|---|---|---|---|---|
| 116.8 | 138.7 | 127.8 | yohimine | 3.7E-07 | 4.8E-08 |
| 112.4 | 76.5 | 94.4 | nor-BNI | 4.3E-10 | 7.2E-11 |
| 100.1 | 101.8 | 101.0 | CTOP | 2.1E-07 | 2.3E-08 |

Compound HCV-3 also was tested for hERG inhibition. Over the concentration range tested (up to 25 micromolar) no dose-response was obtained. Therefore the inhibition $IC_{50}$ was considered as >25 micromolar. There was a hint of some inhibition at the top concentration of 25 micromolar, with 32.5% inhibition observed (insufficient to generate an $IC_{50}$ value). As such, this compound is categorised as having weak or no hERG inhibition. The control compounds behaved as expected in the assay.

Compound HCV-3 also was tested for CYP inhibition, and was found to inhibit CYP2D6, and to weakly inhibit CYP2C19. However, with CYP2C19 the inhibition was too weak to generate an $IC_{50}$ value, and we observed just 36.4% inhibition at the top concentration of 25 micromolar. With CYP2D6, an $IC_{50}$ of 4.2 micromolar was observed. Thus, this compound was considered to be a moderate CYP2D6 inhibitor, and a weak CYP2C19 inhibitor. No inhibition was observed at CYP2B6, CYP2C9, CYP3A4 (with either substrate), CYP2C8 or CYP1A2. The significance of this CYP2D6 inhibition will depend on the levels of the compound *in vivo.*

Compound HCV-3 also was tested in cellular and nuclear receptor functional assays, and the results below and in Figs 5 and 6.

1. SUMMARY

The purpose of this study was to test HCV-3 in cellular and nuclear receptor functional assays.

1.1 Study Design

HCV-3 was tested at 1.0E-05 M.

1.2 Measurements

Cellular agonist effect was calculated as a % of control response to a known reference agonist for each target and cellular antagonist effect was calculated as a % inhibition of control reference agonist response for each target.

1.3 Results

Results showing an inhibition or stimulation higher than 50% are considered to represent significant effects of the test compounds.

Such effects were not observed at any of the receptors studied.

2. COMPOUNDS

2.1 Test Compounds

Manufacturer: TECH LAUNCH ARIZONA

| | |
|---|---|
| Client Compound ID | HCV-3 |
| Compound IC | 100023322-1 |
| Reference Number | - |
| Batch Number | 1 |
| FW | 296.84 |
| MW | 260.38 |
| Purity | - |
| Received Form | Powder |
| Stock Solution | 1.E-02M H20 |
| Flag | - |
| FW: Formula Weight - MW: Molecular Weight | |

2.2 Reference Compounds

In each experiment and if applicable, the respective reference compound was tested concurrently with HCV-3, and the data were compared with historical values determined at Eurofins. The experiment was accepted in accordance with Eurofins validation Standard Operating Procedure.

3. RESULTS

3.1 In Vitro Pharmacology: Cellular and Nuclear Receptor Functional Assays

3.1.1 Agonist Effect: Test Compound Results

% Inhibition of Control Agonist Response

| Compound I.D | Client Compound I.D. | Test Concentration | 1st | 2nd | Mean |
|---|---|---|---|---|---|
| $\alpha_{2B}$(*h*)(agonist effect) | | | | | |
| 100023322-1 | HCV-3 | 1.0E-05M | 9.2 | 5.1 | 7.1 |
| $\delta_2$ (DOP)(agonist effect) | | | | | |
| 100023322-1 | HCV-3 | 1.0E-05M | 4.0 | 4.0 | 4.0 |
| $\kappa$ (KOP)(agonist effect) | | | | | |
| 100023322-1 | HCV-3 | 1.0E-05M | -10.3 | 1.8 | -4.2 |
| $\mu$ (MOP) (*h*) agonist effect | | | | | |
| 100023322-1 | HCV-3 | 1.0E-05M | 28.1 | 38.9 | 33.5 |

3.1.2 Reference Compound Results

| Compound I.D. | EC $_{50}$(M) | nH |
|---|---|---|
| $\alpha_{2B}$ (*h*)(agonist effect) | | |
| dexmedetomidine | 1.3E-08M | n/a |
| $\delta_2$ (DOP)(agonist effect) | | |
| DPDPE | 1.5E-09M | n/a |
| $\kappa$ (KOP)(agonist effect) | | |
| U 50488 | 1.6E-09M | n/a |
| $\mu$ (MOP)(*h*) (agonist effect) | | |
| DAMGO | 4.2E-09M | n/a |

3.1.3 Antagonists Effect: Test Compound Results

% Inhibition of Control Agonist Response

| Compound I.D | Client Compound I.D. | Test Concentration | 1st | 2nd | Mean |
|---|---|---|---|---|---|
| $\alpha_{2B}$ (*h*)(antagonist effect) | | | | | |
| 100023322-1 | HCV-3 | 1.0E-05M | -16.8 | -38.7 | -27.8 |
| $\delta_2$ (DOP)(antagonist effect) | | | | | |
| 100023322-1 | HCV-3 | 1.0E-05M | 0.8 | 5.9 | 3.3 |
| $\kappa$ (KOP)(antagonist effect) | | | | | |
| 100023322-1 | HCV-3 | 1.0E-05M | -12.4 | 23.5 | 5.6 |
| $\mu$ (MOP) (*h*) (antagonist effect) | | | | | |
| 100023322-1 | HCV-3 | 1.0E-05M | -0.1 | -1.8 | -1.0 |

3.1.4 Reference Compound Results

| Compound I.D. | $IC_{50}$ (M) | $K_B$ (M) | nH |
|---|---|---|---|
| $\alpha_{2B}$ (*h*) (antagonist effect) | | | |
| yohimbine | 3.7E-07 M | 4.8E-08 M | n/a |
| $\delta_2$ (DOP) (antagonist effect) | | | |
| naltrindole | 2.7E-10 M | 4.6E-11 M | n/a |
| $\kappa$ (KOP) (antagonist effect) | | | |
| nor-BNI | 4.3E-10 M | 7.2E-11 M | n/a |
| $\mu$ (MOP) (*h*) (antagonist effect) | | | |
| CTOP | 2.1E-07 M | 2.3E-08 M | n/a |

4. *In Vitro* Pharmacology

Results showing a stimulation or an inhibition higher than 50% are considered to represent significant effects of the test compounds, 50% is the most common cutoff value for further investigation (determination of $EC_{50}$ or $IC_{50}$ values from concentration-response curves).

Results showing a stimulation or an inhibition between 25% and 50% are indicative of weak to moderate effects

(in some assays, they may be confirmed by further testing as they are within a range where more inter-experimental variable can occur).

Results showing a stimulation or an inhibition lower than 25% are not considered significant and most attributable to variability of the signal around the control level.

5.1 Experimental Conditions

Minor variations to the experimental protocol described below may have occurred during the testing, they have no impact on the quality of the results obtained.

5.1.1 *In Vitro* Pharmacology: Cellular and Nuclear Receptor Functional Assays

| Assay | Source | Stimulus | Incubation | Measured Component | Detection Method | Bibl. |
|---|---|---|---|---|---|---|
| Receptors | | | | | | |
| $\alpha_{2B}$ (*h*) (agonist effect) | human recombinant (HEK-293 cells) | none (3 µM dexmedeto midine for control | 30 min 37°C | cAMP | HTRF | 915 |
| $\alpha_{2B}$ (*h*) (antagonist effect) | human recombinant (HEK-293 cells) | dexmedeto midine ( 100 nM ) | 30 min 37°C | cAMP | HTRF | 915 |
| $\delta_2$ (DOP) (agonist effect) | NG-10815 cells (endogenous) | none (300 nM DPDPE for control) | 37°C | impedance | Cellular dielectric spectroscopy | 934 |
| $\delta_2$ (DOP) (antagonist effect) | NG-10815 cells (endogenous) | DPDPE ( 10 nM) | 37°C | impedance | Cellular dielectric spectroscopy | 934 |
| κ (KOP) (agonist effect) | rat recombinant (CHO cells) | none (0.3 µM U 50488 for control) | 10 min 37°C | cAMP | HTRF | 819 |
| κ (KOP) (antagonist effect) | rat recombinant (CHO cells) | U 50488 (3 nM) | 10 min 37°C | cAMP | HTRF | 819 |
| µ (MOP) (*h*) (agonist effect) | human recombinant (CHO cells) | none (0.3 µM DAMGO for control | 10 min 37°C | cAMP | HTRF | 260 |
| µ (MOP) (*h*) (antagonist effect) | human recombinant (CHO cells) | DAMGO (20 nM) | 10 min 37°C | cAMP | HTRF | 260 |

5.2.1 *In Vitro* Pharmacology: Cellular and Nuclear Receptor Functional Assays

The results are expressed as a percent of control agonist response

$$\frac{measured\ response}{control\ response} * 100$$

and as a percent inhibition of control agonist response

$$100 - \left(\frac{measured\ response}{control\ response} * 100\right)$$

obtained in the presence of HCV-3.

The $EC_{50}$ values (concentration producing a half-maximal response) and $IC_{50}$ values (concentration causing a half-maximal inhibition of the control agonist response) were determined by non-linear regression analysis of the concentration-response curves generated with mean replicate values using Hill equation curve fitting

$$Y = D + \left[\frac{A-D}{1+(C/C_{50})^{nH}}\right]$$

where Y = response, A = left asymptote of the curve, D = right asymptote of the curve, C = compound concentration, and $C_{50}$=$EC_{50}$ or $IC_{50}$, and nH = slope factor.

This analysis was performed using software developed at Cerep (Hill software) and validated by comparison with data generated by the commercial software SigmaPlot® 4.0 for Windows® (© 1997 by SPSS Inc.).

For the antagonists, the apparent dissociation constants ($K_B$) were calculated using the modified Cheng Prusoff equation

$$K_B = \frac{IC_{50}}{1+(A/EC_{50A})}$$

where A = concentration of reference agonist in the assay, and $EC_{50A}$ = $EC_{50}$ value of the reference agonist.

260. Wang, J.B. et al. (1994), FEBS Lett, 338: 217-222.
819. Avidor-Reiss, T. et al. (1995) FEBS Lett, 361: 70-74.
915. Eason, M.G. et al. (1992) J. Biol. Chem., 267: 15795-15801.
934. Law, P.Y. and Loh, H.H. (1993), Mol Pharmacol., 43: 684-693

Compound HCV-3 was also subjected to AMES testing, and the results reported below:

1. PURPOSE

The purpose of this study was to evaluate the mutagenicity potential of a test article (HCV-3) in the Ames assay using the microplate fluctuation (MPF) method.

2. STUDY CONDITIONS

This study was performed under non-GLP conditions. All work was performed with appropriate local health regulations and ethical approval.

3. EXPERIMENTAL DESIGN

3.1 AMES MFP: Experimental Conditions

| Test Article | Test Concentrations* (μg/mL) | Reference Compounds |
|---|---|---|
| HCV-3 | 31.3, 62.5, 125, 250, 500, 1000 | 2-aminoanthracene<br>2-nitrofluorene<br>4-nitroquinoline N-oxide |
| *The top concentration in the dose response reflects the highest soluble concentration of the test article in Ames assay conditions. | | |

**Experimental Procedure:** Approximately ten million bacteria are exposed in triplicate to test agent (six concentrations), a negative control (vehicle) and a positive control for 90 minutes in medium containing a low concentration of histidine (sufficient for about 2 doublings.) The cultures are then diluted into indicator medium lacking histidine, and dispensed into 48 wells of a 384 well plate (micro-plate format, MPF). The plate is incubated for 48 hr at 37°C, and cells that have undergone a reversion will grow in a well, resulting in a color change in wells with growth. The number of wells showing growth are counted and compared to the vehicle control. An increase in the number of colonies of at least two-fold over baseline (mean +SD of the vehicle control) and a dose response indicates a positive response. An unpaired, one-sided Student's T-test is used to identify conditions that are significantly different from the vehicle control.

Where indicated, S9 fraction from the livers of Aroclor 1254-treated rats is included in the incubation at a final concentration of 4.5%. An NADPH-regenerating system is also included to ensure a steady supply of reducing equivalents.

Strains used in this study:

S. typhimurium TA98: hisD3052, rfa, uvrB / pKM101; detects frame-shift mutations.
S. typhimurium TA 100: hisG45, rfa, uvrB / pKM101; detects base-pair substitutions.

4 . RESULTS AND CONCLUSIONS

4.1 AMES MPF: Data Summary

| Test Article | Test Strain | S9 | AMES Result (Positive/ Negative) | Highest Cone. Tested | Comment |
|---|---|---|---|---|---|
| 2-nitrofluorine + 4-nitroquinoline N-oxide | TA98 | no | Positive | 4 μg/ml + 2 μg/ml | positive control |
| 2-nitrofluorine + 4-nitroquinoline N-oxide | TA100 | no | Positive | 4 μg/ml + 2 μg/ml | positive control |
| aminoanthracene | TA98 | yes | Positive | 5μg/ml | positive control |
| aminoanthracene | TA100 | yes | Positive | 5μg/ml | positive control |
| HCV-3 | TA98 | no | Negative | 1,000 μg/mL | |
| | TA100 | no | Negative | 1,000 μg/mL | |
| | TA98 | yes | Negative | 1,000 μg/mL | |
| | TA100 | yes | Negative | 1,000 μg/mL | |
| *Highest soluble concentration of test article in Ames assay conditions. | | | | | |

**Conclusion:** Test article HCV-3 was assessed for its mutagenic potential in the Ames reverse mutation assay. This test was performed in the absence and presence of S9 metabolic activation. HCV-3 was found to be negative for genotoxicity against both strains used in this study (TA98 and TA100) up to a maximum tested concentration of 1 mg/ml. The positive controls behaved as expected.

**4.2 AMES MPF: Individual Data**

Significant fold increase over baseline values (≥2-fold) are indicated in **bold red**. Significant T-test probabilities (P<0.05) appear red and (P<0.01) are indicated in **bold red**.

HCV-3

| TA 98-S9 | | | | | Assay | | 8/10/2015 |
|---|---|---|---|---|---|---|---|
| Conc. (µg/ml) | n | mean # pos. Wells | Corr. mean | SD | Baseline | Fold increase (over baseline) | t-test p-value (unpaired, 1-sided) |
| 0 | 3 | 1.33 | | 1.53 | 2.86 | | |
| 31.3 | 3 | 1.33 | | 1.53 | | 0.47 | 0.5000 |
| 62.5 | 3 | 1.00 | | 1.00 | | 0.35 | 0.3838 |
| 125 | 3 | 1.00 | | 1.00 | | 0.35 | 0.3838 |
| 250 | 3 | 1.67 | | 2.08 | | 0.58 | 0.4170 |
| 500 | 3 | 0.67 | | 0.58 | | 0.23 | 0.2593 |
| 1000 | 3 | 0.67 | | 0.58 | | 0.23 | 0.2593 |
| Pos. Control | 3 | 26.00 | | 2.65 | | | |

HCV-3

| TA 98+S9 | | | | | Assay | | 8/10/2015 |
|---|---|---|---|---|---|---|---|
| Conc. (µg/ml) | n | mean # pos. Wells | Corr. mean | SD | Baseline | Fold increase (over baseline) | t-test p-value (unpaired, 1-sided) |
| 0 | 3 | 4.00 | | 1.00 | 5.00 | | |
| 31.3 | 3 | 5.67 | | 2.52 | | 1.13 | 0.1732 |
| 62.5 | 3 | 6.67 | | 2.31 | | 1.33 | 0.0702 |
| 125 | 3 | 6.00 | 3.61 | | 1.20 | 0.2035 | |
| 250 | 3 | 6.00 | 4.36 | | 1.20 | 0.2409 | |
| 500 | 3 | 3.67 | 2.08 | | 0.73 | 0.4075 | |
| 1000 | 3 | 5.00 | 4.00 | | 1.00 | 0.3480 | |
| Pos. Control | 3 | 34.33 | 8.33 | | | | |

HCV-3

| TA 100-S9 | | | | | Assay | | 8/10/2015 |
|---|---|---|---|---|---|---|---|
| Conc. (µg/ml) | n | mean # pos. Wells | Corr. mean | SD | Baseline | Fold increase (over baseline) | t-test p-value (unpaired, 1-sided) |
| 0 | 3 | 5.67 | | 1.53 | 7.19 | | |
| 31.3 | 3 | 2.33 | | 0.58 | | 0.32 | 0.0121 |
| 62.5 | 3 | 3.67 | | 2.08 | | 0.51 | 0.1254 |
| 125 | 3 | 3.67 | | 2.52 | | 0.51 | 0.1523 |
| 250 | 3 | 2.67 | | 0.58 | | 0.37 | 0.0167 |
| 500 | 3 | 2.67 | | 0.58 | | 0.37 | 0.0167 |
| 1000 | 3 | 2.33 | | 0.58 | | 0.32 | 0.0121 |
| Pos. Control | 3 | 47.67 | | 0.58 | | | |

HCV-3

| TA 100 +S9 | | | | | Assay | | 8/10/2015 |
|---|---|---|---|---|---|---|---|
| Conc. (μg/ml) | n | mean # pos. Wells | Corr. mean | SD | Baseline | Fold increase (over baseline) | t-test p-value (unpaired, 1-sided) |
| 0 | 3 | 2.00 | | 1.73 | 3.73 | | |
| 31.3 | 3 | 2.67 | | 0.58 | | 0.71 | 0.2807 |
| 62.5 | 3 | 3.33 | 1.53 | | 0.89 | 0.1870 | |
| 125 | 3 | 3.33 | 2.52 | | 0.89 | 0.2459 | |
| 250 | 3 | 4.33 | 1.53 | | 1.16 | 0.0775 | |
| 500 | 3 | 4.00 | 1.00 | | 1.07 | 0.0792 | |
| 1000 | 3 | 4.33 | 1.15 | | 1.16 | 0.0621 | |
| Pos. Control | 3 | 25.33 | 12.10 | | | | |

5. REFERENCE

Umbuzeiro, G. et al. (2010). "Comparison of the Salmonella/microsome micro-suspension assay with the new microplate fluctuation protocol for testing the mutagenicity of environmental samples". Environ. Mol. Mutagen. 51 (1):31-38.

In summary, the compound HCV-3 was negative for genotoxicity against both strains used in this assay (TA98 and TA 100) up to a maximum tested concentration of 1mg/mL, in both the absence and presence of S9 metabolic activation. The assay controls behaved as expected.

Compound HCV-3 also was subjected to in vitro metabolic disposition in mouse, rat, monkey and human microsomes. The test compound was incubated with pooled liver microsomes, since drip stability in liver microsomes can be predictive of drug stability *in vivo.* Aliquots were taken at 0, 5, 15, 30 and 45 minutes and quenched immediately. The samples were extracted and analyzed by LC-MS/MS. Compound HCV-3 was observed to have low clearance in human, monkey and mouse microsomes, and moderate clearance in rat.

Compound HCV-3 also was subjected to MDCK permeability assay. The compound was observed to be highly permeable in the MDCK assay. There was a slight difference between the plus and minus inhibitor data in terms of the efflux ratio obtained (1.48 minus inhibitor, versus 0.929 plus inhibitor). A ratio of greater than 2 generally indicates that efflux, i.e., blood brain barrier permeability, is occurring. The control compounds behaved as expected, with prazosin (a P-gp substrate) showing efflux in the absence of Cyclosporin A, which was inhibited in its presence.

Various derivatives of the above compounds with potential opiod and alpha antagonist activity were prepared as follows, and characterized by NMR and mass-spec as reported below.

**Protocols for the synthesis of substituted 1-arylethyl-4-acylaminopiperidine derivatives**

***Synthesis of 1-benzylpiperidin-4-one oxime***

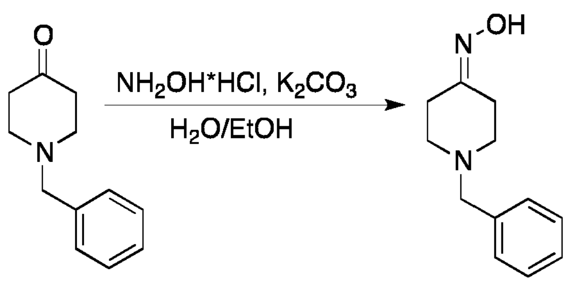

28.35 g (1 equiv., 0.15 mol) of 1-benzylpiperidin-4-one was dissolved in 60 mL of EtOH and then cooled to 0 °C using an ice bath. A solution containing 20.85 g (2 equiv., 0.30 mol) of hydroxylamine hydrochloride dissolved in 75 mL of $H_2O$ was prepared and then added dropwise to the reaction mixture followed by dropwise addition of a solution containing 20.7 g (1 equiv., 0.15 mol) of $K_2CO_3$ dissolved in 75 mL of $H_2O$. The reaction mixture was then brought to room temperature and stirred overnight. The EtOH was then removed via rotary evaporation and the reaction mixture was then cooled in an ice bath to allow the product to crystallize out of solution. The product was filtered and washed several times with $H_2O$ and recrystallized in EtOH. Yield: **27.78 g** (70.17%).

***Synthesis of 1-benzylpiperidin-4-amine***

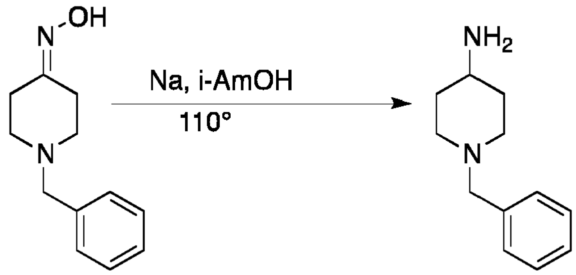


A solution containing 6.12 g (1 equiv., 0.03 mol) of 1-benzylpiperidin-4-one oxime dissolved in 90 mL of iso-amyl alcohol was prepared and heated to approximately 110 °C. 6.9 g (10 equiv., 0.3 mol) of Na metal was then added slowly to the reaction mixture. After addition of Na, the reaction mixture was allowed to cool to room temperature and stirred until the reaction mixture turned into a thick slurry. The slurry was dissolved in 50 mL of ethyl acetate and 25 mL of $H_2O$. The organic layer was separated and washed with $H_2O$ (2 × 20 mL) followed by drying over anhydrous magnesium sulfate. The solvent was removed via rotary evaporation, resulting in a yellow oil. The crude product was purified via column chromatography utilizing silica gel and a DCM:MeOH solvent system in a ratio of 4:1 with an additional 1% of $Et_3N$. Yield: **3.7 g** (64%).

***Synthesis of N*-(1-benzylpiperidin-4-yl)propionamide**

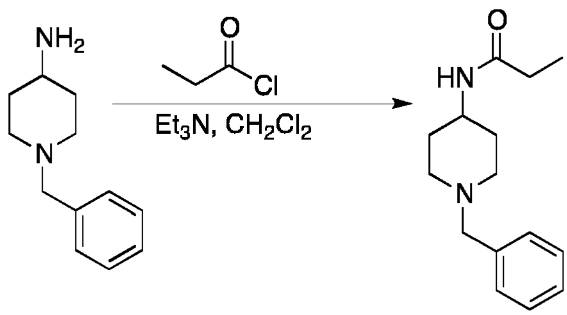


A solution of 3.7 g of 1-benzylpiperidin-4-amine (1 equiv., 0.019 mol) dissolved in 45 mL of dry dichloromethane was prepared followed by the addition of 5 mL of $Et_3N$ (2.6 equiv., 0.05 mol). The reaction mixture was then cooled to 0 °C using an ice bath, and then 2.17 mL (1.3 equiv., 0.025 mol) of propionyl chloride dissolved in 10 mL of dry dichloromethane was added dropwise to the reaction mixture. The reaction mixture was then warmed to room temperature and stirred overnight. Once the reaction was complete, 4 mL of $NH_4OH$ and 45 mL of $H_2O$ were added to the reaction mixture. The organic layer was separated, and the aqueous layer was washed with dichloromethane (3 × 20 mL) followed by $NaHCO_3$ solution and brine. The organic extracts were dried over anhydrous magnesium sulfate and the solvent was removed via rotary evaporation, resulting in a white solid. The product was washed with hexanes to obtain an analytically pure sample. Yield: **2.8 g** (72%)

***Synthesis of N-*(piperidin-4-yl)propionamide**

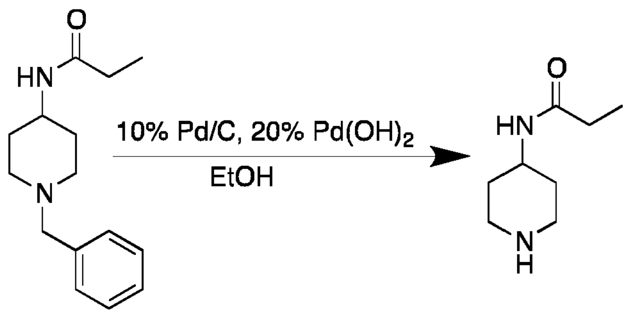


0.7 g of N (1-benzylpiperidin-4-yl)propionamide (1 equivalent, 0.003 moles) were added to a parr hydrogenation flask and dissolved in 30 mL of EtOH. The solution was then degassed with argon for 30 min followed by the addition of 0.07 g of 10% Pd/C (0.2 equiv., 6.58 $\times$ $10^{-4}$ mol) and 0.07 g of 20% $Pd(OH)_2$ (0.17 equiv., 4.98 $\times$ $10^{-4}$ mol). The black solution was then degassed with argon for an additional 15 min. The reaction mixture was then charged with 50 psi of $H_2$ gas and shaken for 24 h. The product was filtered through celite and the solvent was removed via rotary evaporation. No further purification was required. Yield: **0.467 g** (99%)

***Synthesis of methyl 3-(4-propionamidopiperidin-1-yl)propanoate (CRA5)***

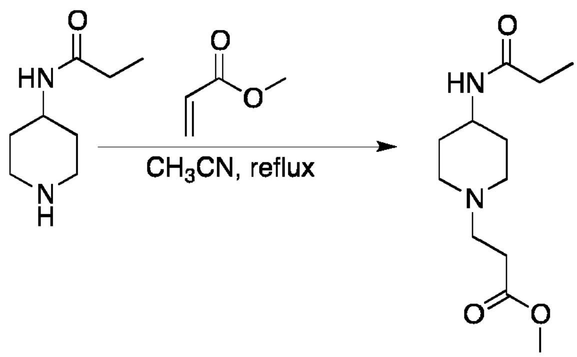


0.1 g of N (piperidin-4-yl)propionamide (1 equiv., 5.26 $\times$ $10^{-4}$ moles) was dissolved in 2 mL of dry acetonitrile followed by the addition of 0.071 mL of methyl acrylate (1.5 equiv., 7.89 $\times$ $10^{-4}$ mol). The reaction mixture was refluxed overnight. The solvent was removed via rotary evaporation. The crude product was purified by washing with hexanes followed by drying under high vacuum. Yield: **0.90 g** (71%)

***Synthesis of N-(1-(2-(thiophen-2-yl)ethyl)piperidin-4-yl)propionamide (CRAS1)***

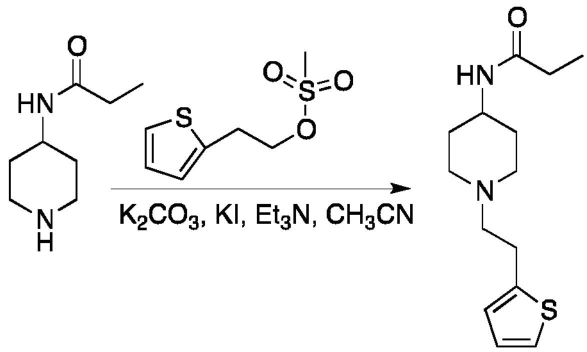


0.1 g of *N*-(piperidin-4-yl)propionamide (1 equiv., 6.40 × $10^{-4}$ mol), 0.145 g of 2-(thiophen-2-yl)ethyl methanesulfonate (1.1 equiv., 7.04 × $10^{-4}$ moles), 0.097 g of $K_2CO_3$ (1.1 equiv., 7.04 × $10^{-4}$ mol), 0.032 g of KI (1.92 × $10^{-4}$ mol), and 0.178 mL of $Et_3N$ (2 equiv., 1.28 × $10^{-3}$ mol) were added to a round bottom flask and dissolved in 5 mL of dry acetonitrile. The reaction mixture was stirred and refluxed overnight. The solvent was then removed via rotary evaporation followed by the addition of $H_2O$. The mixture was extracted with ethyl acetate (3 × 5 mL), and the organic extracts were combined and dried over anhydrous magnesium sulfate. The solvent was removed via rotary evaporation. The crude product was washed with hexanes to obtain an analytically pure sample. Yield: **0.101 g** (60%).

***Synthesis of 1-phenethylpiperidin-4-one oxime***

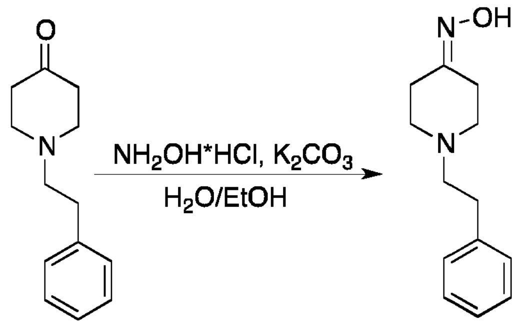


28.35 g (1 equivalent, 0.14 mol) of 1-benzylpiperidin-4-one were dissolved in 60 mL of EtOH and then cooled to 0° C using an ice bath. A solution containing 19.46 g (2 equivalents, 0.28 mol) of hydroxylamine hydrochloride dissolved in 75 mL of $H_2O$ was prepared and then added dropwise to the reaction mixture followed by dropwise addition of a solution containing 19.35 g (1 equivalent, 0.14 mol) of $K_2CO_3$ dissolved in 75 mL of $H_2O$. The reaction mixture was then brought to room temperature and stirred overnight. The EtOH was then removed via rotary evaporation and the reaction mixture was then cooled in an ice bath to allow the product to crystallize out of solution. The product was filtered and washed several times with $H_2O$ and recrystallized in EtOH. Yield: **25.60 g** (83.77%).

***Synthesis of 1-phenethylpiperidin-4-amine***

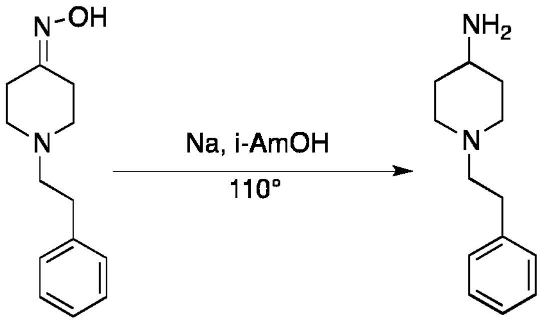


A solution containing 6.00 g (1 equiv., 0.027 mol) of 1-phenethylpiperidin-4-one oxime dissolved in 90 mL of iso-amyl alcohol was prepared and heated to approximately 110 °C. 6.21 g (10 equiv., 0.27 mol) of Na metal was then added slowly to the reaction mixture. After addition of Na, the reaction mixture was allowed to cool to room temperature and stirred until the reaction mixture turned into a thick slurry. The slurry was dissolved in 50 mL of ethyl acetate and 25 mL of $H_2O$. The organic layer was separated and washed with $H_2O$ ($2 \times 20$ mL) followed by drying over anhydrous magnesium sulfate. The solvent was removed via rotary evaporation, resulting in a yellow oil. The crude product was purified via column chromatography utilizing silica gel and a DCM:MeOH solvent system in a ratio of 4:1 containing an additional 1% of $Et_3N$.

***Synthesis of N-(1-phenethylpiperidin-4-yl)furan-2-carboxamide (CRA8)***

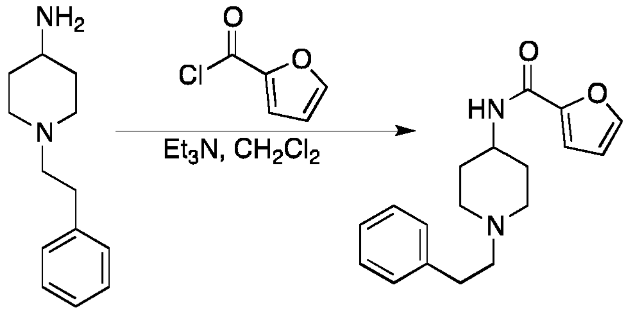


A solution of 0.1 g of 1-phenethylpiperidin-4-amine (1 equiv., $4.89 \times 10^{-4}$ mol) dissolved in 2 mL of dry dichloromethane was prepared followed by the addition of 0.178 mL (2.6 equiv., $1.27 \times 10^{-3}$ moles) of $Et_3N$. The reaction mixture was then cooled to 0 °C using an ice bath, and then 0.063 mL (1.3 equiv., $6.36 \times 10^{-4}$ mol) of 2-furoyl chloride dissolved in 0.25 mL of dry dichloromethane was added dropwise to the reaction mixture. The reaction mixture was then warmed to room temperature and stirred overnight. Once the reaction was complete, 4 mL of $NH_4OH$ and 45 mL of $H_2O$ were added to the reaction mixture. The organic layer was separated, and the aqueous layer was washed with dichloromethane ($3 \times 5$ mL) followed by $NaHCO_3$ solution and brine. The organic extracts were dried over anhydrous magnesium sulfate and the solvent was removed via rotary evaporation, resulting in a white solid. The product was washed with hexanes to obtain an analytically pure sample. Yield: **0.0845 g** (58.3 %).

***Synthesis of N-(1-phenethylpiperidin-4-yl)furan-3-carboxamide (CRA9)***

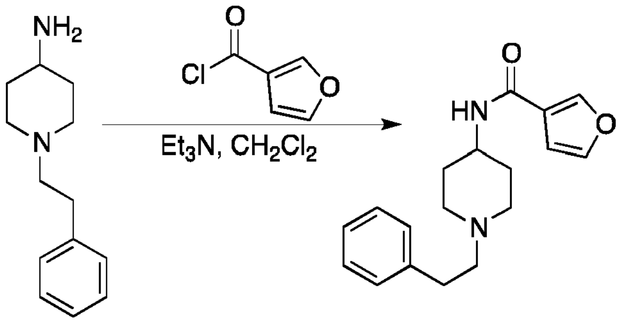


A solution of 0.1 g of 1-phenethylpiperidin-4-amine (1 equiv., 4.89 × $10^{-4}$ mol) dissolved in 2 mL of dry dichloromethane was prepared followed by the addition of 0.178 mL (2.6 equiv., 1.27 × $10^{-3}$ mol) of $Et_3N$. The reaction mixture was then cooled to 0 °C using an ice bath, and then 0.063 mL (1.3 equiv., 6.36 × $10^{-4}$ mol) of 3-furoyl chloride dissolved in 0.25 mL of dry dichloromethane was added dropwise to the reaction mixture. The reaction mixture was then warmed to room temperature and stirred overnight. Once the reaction was complete, 4 mL of $NH_4OH$ and 45 mL of $H_2O$ were added to the reaction mixture. The organic layer was separated, and the aqueous layer was washed with dichloromethane (3 × 5 mL) followed by $NaHCO_3$ solution and brine. The organic extracts were dried over anhydrous magnesium sulfate and the solvent was removed via rotary evaporation, resulting in a white solid. The product was washed with hexanes to obtain an analytically pure sample. Yield: **0.104 g** (72%).

***Synthesis of 8-bromo-N-(1-phenethylpiperidin-4-yl)octanamide (CRA10)***

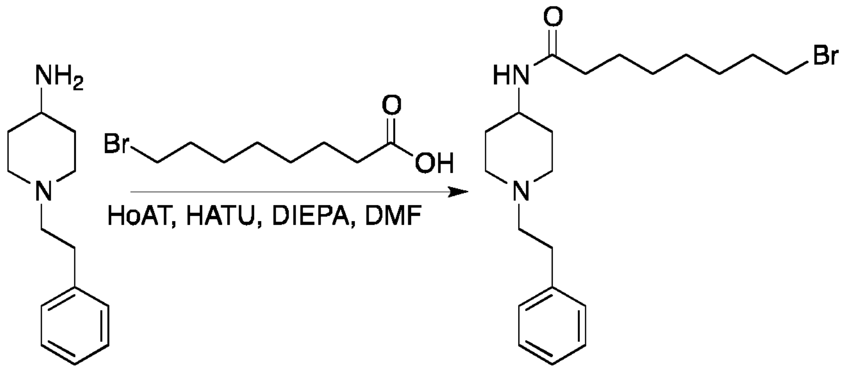


A solution of 0.101 g of 1-phenethylpiperidin-4-amine (1.1 equiv., 4.93 × $10^{-4}$ mol), 0.1 g of 8-bromooctanoic acid (1.0 equiv., 4.48 × $10^{-4}$ mol), 0.170 g of HATU (1.0 equiv., 4.48 × $10^{-4}$ mol), 0.061 g of HOAt (1.0 equiv., 4.48 × $10^{-4}$ mol), and 0.314 mL of DIEPA (4.0 equiv., 0.0018 mol) in dry DMF was prepared. The reaction mixture was stirred at room temperature overnight. The reaction mixture was then quenched with 0.5 M $KHSO_4$ solution followed by the addition of dichloromethane. The organic and aqueous layers were separated, and the aqueous layer was extracted with dichloromethane (3 × 5 mL) followed by washing with NaHCOs solution and Brine. The organic extracts were then dried over anhydrous magnesium sulfate.

***Synthesis of N-(1-phenethylpiperidin-4-yl)benzamide (CRA11)***

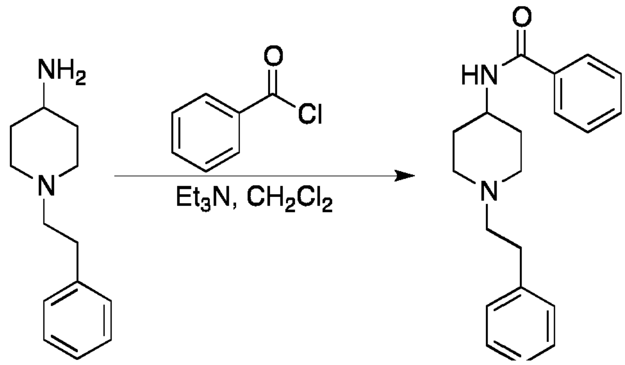


A solution of 0.1 g of 1-phenethylpiperidin-4-amine (1 equiv., $4.89 \times 10^{-4}$ mol) dissolved in 2 mL of dry dichloromethane was prepared followed by the addition of 0.178 mL (2.6 equiv., $1.27 \times 10^{-3}$ mol) of $Et_3N$. The reaction mixture was then cooled to 0 °C using an ice bath, and then 0.074 mL (1.3 equiv., $6.36 \times 10^{-4}$ mol) of 3-furoyl chloride dissolved in 0.25 mL of dry dichloromethane was added dropwise to the reaction mixture. The reaction mixture was then warmed to room temperature and stirred overnight. Once the reaction was complete, 4 mL of $NH_4OH$ and 45 mL of $H_2O$ were added to the reaction mixture. The organic layer was separated, and the aqueous layer was washed with dichloromethane ($3 \times 5$ mL) followed by $NaHCO_3$ solution and brine. The organic extracts were dried over anhydrous magnesium sulfate and the solvent was removed via rotary evaporation, resulting in a white solid. The product was washed with hexanes to obtain an analytically pure sample.

***Synthesis of 2-(thiophen-2-yl)ethyl methanesulfonate***

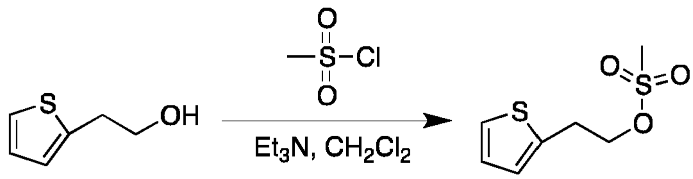


2.6 mL of 2-(thiophen-2-yl)ethanol (1 equiv., 0.023 mol) was dissolved in 45 mL of dry dichloromethane followed by the addition of 3.63 mL of $Et_3N$ (1.13 equiv., 0.026 mol). The reaction mixture was stirred at room temperature for 1 h. It was then cooled to -5 °C using an ice bath and solid NaCl. Once cooled, 1.92 mL of methanesulfonyl chloride was added dropwise over the course of 10 min. The reaction mixture was then warmed to room temperature and stirred for 1 h. Once the reaction was complete, 30 mL of $NaHCO_3$ solution was added followed by separation of the organic and aqueous layers. The aqueous layer was extracted with dichloromethane ($3 \times 30$ mL). The combined organic extracts were dried over anhydrous magnesium sulfate and the solvent was removed via rotary evaporation, resulting in a brown oil. No further purification was required.

**NMR ($^1H$ & $^{13}C$) & mass-spec data of novel substituted 1-arylethyl-4-acylaminopiperidine derivatives (total six compounds)**

| Compound | Molecular Formula | Structure | $R_f$ | Exact Mass | Observed Mass $[M+H]^+$ | Yield |
|---|---|---|---|---|---|---|
| **CRA5** | $C_{12}H_{22}N_2O_3$ | 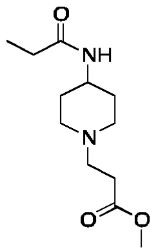 | 0.72 (20% MeOH in DCM) | 242.16 | 243.3 | 90 mg (70.7%) |
| **CRA8** | $C_{18}H_{22}N_2O_2$ | 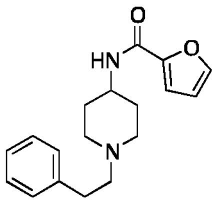 | 0.825 (4:1 MeOH DCM) | 298.17 | 299.3 | 84.5 mg (58.3%) |
| **CRA9** | $C_{18}H_{22}N_2O_2$ | 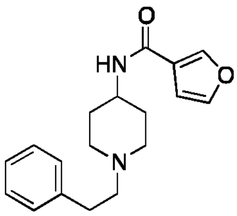 | 0.757 (4:1 MeOH DCM | 298.17 | 299.3 | 104.3 mg(71.9 %) |
| **CRAS1** | $C_{14}H_{22}N_2OS$ | 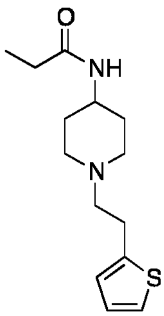 | | 266.15 | 267.73 | 101.4 mg (60%) |
| **CRA10** | $C_{21}H_{33}BrN_2O$ | 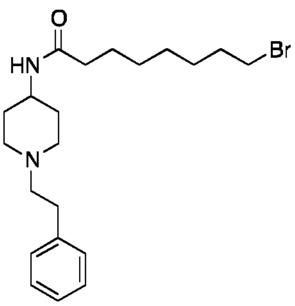 | 0.90 | 408.18 | 409.19 411.19 | 262 mg (130%) |

(continued)

| Compound | Molecular Formula | Structure | $R_f$ | Exact Mass | Observed Mass $[M+H]^+$ | Yield |
|---|---|---|---|---|---|---|
| **CRA11** | $C_{20}H_{24}N_2O$ | 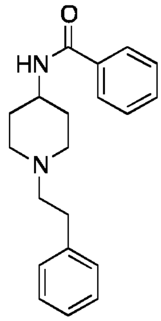 | 0.875 | 308.19 | 309.2 | 132 mg (87%) |

**CRA5 NMR data:** **$^1$H NMR** (400 MHz, $CDCl_3$) δ 1.14 (t, J= 7.59, 7.59 Hz, 3H), 1.41 (dtd, J=3.70, 11.10, 11.13, 12.61 Hz, 2H), 1.91 (m, 2H), 2.17 (m, 4 H), 2.49 (m, 2H), 2.68 (m, 2H), 2.81 (m, 2H), 3.67 (s, 3H), 3.78 (dddd, J=4.29, 4.36, 11.92, 15.26, 1H), 5.25 (d, J= 7.96 Hz, 1H). **$^{13}$C NMR** (100 MHz, $CDCl_3$) δ 10.02, 29.99, 32.41, 46.40, 51.76, 52.25, 55.63, 173.14, 174.05.

**CRA8 NMR data:** **$^1$H NMR** (400 MHz, $CDCl_3$) δ 1.63 (d, J=11.93 Hz, 2H), 2.05 (d, J=12.33 Hz, 2H), 2.26 (t, J=11.35, 11.35 Hz, 2H), 2.64 (dd, J=6.16, 10.21 Hz, 2H), 2.83 (dd, J=6.12, 10.24 Hz, 2H), 2.99 (d, J=12.01 Hz, 2H), 3.98 (m, 1H), 6.21 (d, J=8.20 Hz, 1H), 6.49 (dd, J=1.79, 3.47 Hz, 1H), 7.10 (dd, J=0.84, 3.47 Hz, 1H), 7.24 (m, 5H), 7.43 (dd, 0.84, 1.77 Hz, 1H). **$^{13}$C NMR** (100 MHz, $CDCl_3$) δ 32.67, 34.17, 46.27, 46.61, 52.76, 60.88, 112.60, 114.57, 126.56, 128.87, 129.13, 140.58, 144.16, 148.48, 158.13.

**CRA9 NMR data:** **$^1$H NMR** (400 MHz, $CDCl_3$) δ 1.63 (m, 2H), 2.06 (d, J=11.35 Hz, 2H), 2.26 (m, J=11.35, 2H), 2.65 (m, 2H), 2.84 (m, 2H), 3.02 (d, J=11.86 Hz, 2H), 3.99 (m, 1H), 5.65 (d, J=7.99 Hz, 1H), 6.59 (dd, J=0.91, 1.93 Hz, 1H), 7.24 (m, 5H), 7.42 (dd, 1.58, 1.91 Hz, 1H), 7.91 (dd, J=0.90, 1.59 Hz, 1H). **$^{13}$C NMR** (100 MHz, $CDCl_3$) δ 32.04, 33.49, 46.43, 52.35, 60.26, 108.25, 122.66, 126.19, 128.46, 128.68, 139.88, 143.72, 144.69, 161.95

**CRA1S NMR data:** **$^1$H NMR** (400 MHz, $CDCl_3$) δ 1.15 (t, J=7.58, 7.58 Hz, 3H), 1.47 (m, 2H), 1.95 (m, 2H), 2.18 (m, 4H), 2.64 (dd, J=6.87, 8.62 Hz, 2H), 2.90 (m, 2H), 3.00 (m, 2H), 3.82 (dddd, J=4.20, 8.31, 10.86, 15.17 Hz, 1H), 5.32 (d, J=7.95 Hz, 1H), 6.81 (dq, J=1.02, 1.02, 1.02, 3.20 Hz, 1H), 6.91 (dd, J=3.39, 5.14 Hz, 1H), 7.11 (dd, J=1.21, 5.13 Hz, 1H). **$^{13}$C NMR** (100 MHz, $CDCl_3$) δ 10.04, 28.06, 30.03, 32.15, 46.52, 52.42, 59.98, 123.62, 124.71, 126.69, 142.87, 173.15.

**CRA10 NMR data:** **$^1$H NMR** (400 MHz, $CDCl_3$) δ 1.32-2.34 (m, 14H), 2.82-3.01 (m, 11H), 3.16 (dd, J=6.53, 10.91 Hz, 1H), 3.49 (d, J=11.91 Hz, 1H), 4.63 (dt, J=5.61, 5.61, 8.76 Hz, 1H), 7.24 (m, 4H), 7.42 (dd, J=4.46, 8.37, 1H), 8.02 (m, 1H). **$^{13}$C NMR** (100 MHz, $CDCl_3$) δ 25.17, 25.45, 27.85, 28.70, 28.75, 31.46, 36.55, 38.61, 81.47, 120.73, 128.66, 128.86, 129.19, 151.29, 162.71, 173.62.

**CRA11 NMR data:** **$^1$H NMR** (400 MHz, $CDCl_3$) δ 1.35 (t, J=7.29, 7.29 Hz, 2H), 1.64 (qd, J=3.80, 11.29, 11.29, 11.35 Hz, 2H), 2.08 (m, 2H), 2.27 (td, J=2.57, 11.61, 11.65 Hz, 2H), 2.64 (m, 2H), 2.83 (m, 2H), 3.00 (m, 3H), 4.04 (dddd, J=4.28, 8.29, 10.85, 15.24 Hz, 1H), 6.04 (d, J=7.94 Hz, 1H), 7.25 (m, 5H), 7.44 (m, 3H), 7.75 (m, 2H). **$^{13}$C NMR** (100 MHz, $CDCl_3$) δ 32.22, 33.71, 45.83, 46.97, 52.38, 60.42, 114.25, 126.12, 126.86, 128.42, 128.56, 128.68, 131.42, 134.75, 140.11, 166.88

**Claims**

**1.** A compound having the formula:

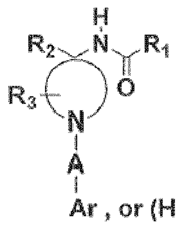

Ar , or (HetAr), or $COOR_5$, or $COON(R_6)_2$ (III)

wherein

$R^1$ is substituted or unsubstituted $C_{1-10}$ alkyl, alkenyl, or alkynyl, or substituted or unsubstituted aryl or heteroaryl;
$R^2$ is H, $-CH_2O-C_{1-4}$ alkyl; $COO-C_{1-4}$ alkyl; $-CONR_4$;
$R^3$ is H, substituted or unsubstituted $C_1$-$C_{10}$ alkyl, alkynyl, or substituted or unsubstituted aryl or hetaryl;
A is substituted or unsubstituted $C_{1-10}$ alkylene, alkynylene;
Ar or HetAr is substituted or unsubstituted monocyclic or polycyclic aromatic or heteroaromatic moiety;
$COOR_5$, or $CON(R_6)_2$, where $R_5$ and $R_6$ are H, substituted or unsubstituted $C_1$-$C_{10}$ alkyl, alkynyl, or substituted or unsubstituted aryl or hetaryl;
the central nitrogen-containing ring is a substituted or un substituted 5- to 7-membered heterocyclic ring; and pharmaceutically acceptable salts of said compound; for use in the treament of a clinical condition associated with opioid abuse, a development of opioid tolerance and dependence, opioid-induced constipation, alcohol abuse, opioid abuse, cocaine abuse, depression, opioid induced immune response depression, opioid-overdose-induced respiratory depression, nicotine withdrawal symptoms, obesity, psychosis, dyskinesia associated with Parkinson's disease, Raynaud's disease, scleroderma or a combination thereof, through modulation of $\mu$-, $\delta$-, k-opioid receptors and $\alpha$2-adrenoreceptors.

**2.** The compound for use according to claim 1, wherein the compound belongs to N-(1-arylethylpiperidin-4-yl)acylamides.

**3.** The compound for use according to claim 1, wherein the compound is N-(1-phenethylpiperidin-4-yl)propionamide.

**4.** The compound for use according to claim 1, wherein the compound is N-(1-phenethylpiperidin-4-yl)propionamide oxalate.

**5.** The compound for use according to claim 1, which is a compound of the formula:

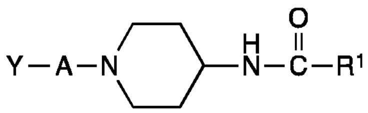

or a pharmaceutically acceptable salt thereof, wherein

$R^1$ is optionally substituted $C_{1-10}$ alkyl, optionally substituted aryl, or optionally substituted heteroaryl;
A is $C_{1-10}$ alkylene; and
Y is optionally substituted aryl, optionally substituted heteroaryl, or a moiety of the formula $-C(=O)-X^1$, wherein $X^1$ is $-OR^5$ or $-NR^6R^6$, wherein each of $R^5$ and $R^6$ is H or $C_{1-10}$ alkyl.

**6.** The compound for use according to claim 5, wherein A is ethylene.

**7.** The compound for use according to claim 5, wherein Y is selected from the group consisting of phenyl, thiophen-2-yl, and a moiety of the formula $-C(=O)-OR^5$, wherein $R^5$ is $C_{1-10}$ alkyl.

**8.** The compound for use according to claim 1 selected from the group consisting of:

N (1-benzylpiperidin-4-yl)propionamide;
methyl 3-(4-propionamidopiperidin-1-yl)propanoate;
N (1-phenethylpiperidin-4-yl)furan-2-carboxamide;

N (1-phenethylpiperidin-4-yl)furan-3-carboxamide;
N (1-(2-(thiophen-2-yl)ethyl)piperidin-4-yl)propionamide;
8-bromo-*N*-(1-phenethylpiperidin-4-yl)octanamide; and
*N*-(1-phenethylpiperidin-4-yl)benzamide.

**9.** The compound for use according to claim 1, wherein said clinical condition comprises dyskinesia associated with Parkinson's disease.

**10.** A compound having the formula (III)

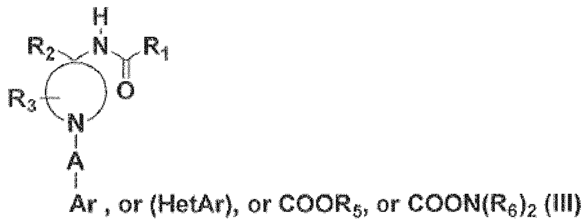

(III)

wherein

$R^1$ is substituted or unsubstituted $C_{1-10}$ alkyl, alkenyl, or alkynyl, or substituted or unsubstituted aryl or heteroaryl;
$R^2$ is H, $-CH_2O-C_{1-4}$ alkyl; $COO-C_{1-4}$ alkyl; $-CONR_4$;
$R^3$ is H, substituted or unsubstituted $C_1$-$C_{10}$ alkyl, alkynyl, or substituted or unsubstituted aryl or hetaryl;
A is substituted or unsubstituted $C_{1-10}$ alkylene, alkynylene;
Ar or HetAr is substituted or unsubstituted monocyclic or polycyclic aromatic or heteroaromatic moiety;
$COOR_5$, or $CON(R_6)_2$, where $R_5$ and $R_6$ are H, substituted or unsubstituted $C_1$-$C_{10}$ alkyl, alkynyl, or substituted or unsubstituted aryl or hetaryl;
the central nitrogen-containing ring is a substituted or un substituted 5- to 7-membered heterocyclic ring;

which is selected from the group consisting of:

*N*-(1 -phenethylpiperidin-4-yl)propionamide;
*N*-(1-phenethylpiperidin-4-yl)propionamide oxalate;
*N*-(1-benzylpiperidin-4-yl)propionamide;
methyl 3-(4-propionamidopiperidin-1-yl)propanoate;
*N*-(1-phenethylpiperidin-4-yl)furan-2-carboxamide;
*N*-(1-phenethylpiperidin-4-yl)furan-3-carboxamide;
*N*-(1-(2-(thiophen-2-yl)ethyl)piperidin-4-yl)propionamide; and
8-bromo-*N*-(1-phenethylpiperidin-4-yl)octanamide.

## Patentansprüche

**1.** Eine Verbindung mit der Formel:

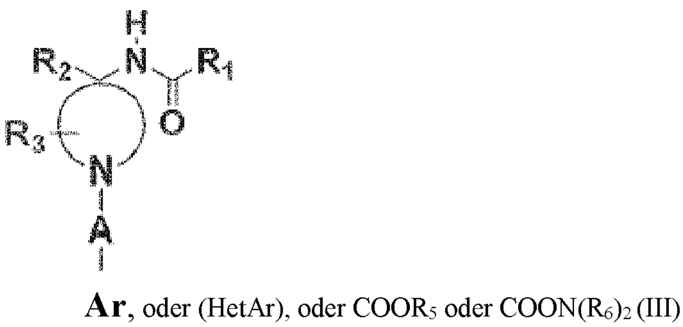

(III)

wobei

$R^1$ substituiertes oder unsubstituiertes $C_{1-10}$-Alkyl, -Alkenyl oder -Alkinyl, oder substituiertes oder unsubstituiertes Aryl oder Heteroaryl ist;
$R^2$ H, $-CH_2O-C_{1-4}$-Alkyl; $COO-C_{1-4}$-Alkyl; $-CONR_4$ ist;
$R^3$ H, substituiertes oder unsubstituiertes $C_1$-$C_{10}$-Alkyl, -Alkinyl oder substituiertes oder unsubstituiertes Aryl oder Hetaryl ist;
A substituiertes oder unsubstituiertes $C_{1-10}$-Alkylen, -Alkinylen ist;
Ar oder HetAr ein substituiertes oder unsubstituiertes monocyclisches oder polycyclisches aromatisches oder heteroaromatisches Teil ist;
$COOR_5$ oder $CON(R_6)_2$, wobei $R_5$ und $R_6$ H, substituiertes oder unsubstituiertes $C_1$-$C_{10}$-Alkyl, -Alkinyl oder substituiertes oder unsubstituiertes Aryl oder Hetaryl sind;
der zentrale stickstoffhaltige Ring ein substituierter oder unsubstituierter 5- bis 7-gliedriger heterocyclischer Ring ist; und pharmazeutisch akzeptable Salze der Verbindung; zur Verwendung bei der Behandlung eines klinischen Zustands, der verbunden ist mit Opioidmissbrauch, einer Entwicklung von Opioidtoleranz und -abhängigkeit, durch Opioide induzierter Verstopfung, Alkoholmissbrauch, Opioidmissbrauch, Kokainmissbrauch, Depression, durch Opioide induzierter Immunantwortdepression, durch Opioidüberdosis induzierter Atemdepression, Nikotinentzugssymptomen, Fettleibigkeit, Psychose, Dyskinesie, die mit Parkinson-Krankheit verbunden ist, Raynaud-Krankheit, Sklerodermie oder einer Kombination davon, durch Modulation von $\mu$-, $\delta$-, $\kappa$-Opioidrezeptoren und $\alpha$2-Adrenorezeptoren.

2. Die Verbindung zur Verwendung nach Anspruch 1, wobei die Verbindung zu den N-(1-Arylethylpiperidin-4-yl)acylamiden gehört.

3. Die Verbindung zur Verwendung nach Anspruch 1, wobei die Verbindung N-(1-Phenethylpiperidin-4-yl)propionamid ist.

4. Die Verbindung zur Verwendung nach Anspruch 1, wobei die Verbindung N-(1-Phenethylpiperidin-4-yl)propionamidoxalat ist.

5. Die Verbindung zur Verwendung nach Anspruch 1, welche eine Verbindung der Formel:

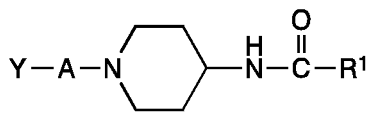

oder ein pharmazeutisch akzeptables Salz davon ist, wobei

$R^1$ wahlweise substituiertes $C_{1-10}$-Alkyl, wahlweise substituiertes Aryl oder wahlweise substituiertes Heteroaryl ist;
A $C_{1-10}$-Alkylen ist; und
Y wahlweise substituiertes Aryl, wahlweise substituiertes Heteroaryl, oder ein Teil der Formel -C(=O)-X' ist, wobei $X^1$ $-OR^5$ oder $-NR^6R^6$ ist, wobei jedes von $R^5$ und $R^6$ H oder $C_{1-10}$-Alkyl ist.

6. Die Verbindung zur Verwendung nach Anspruch 5, wobei A Ethylen ist.

7. Die Verbindung zur Verwendung nach Anspruch 5, wobei Y ausgewählt ist aus der Gruppe bestehend aus Phenyl, Thiophen-2-yl und einem Teil der Formel $-C(=O)-OR^5$, wobei $R^5$ $C_{1-10}$-Alkyl ist.

8. Die Verbindung zur Verwendung nach Anspruch 1, ausgewählt aus der Gruppe bestehend aus:

*N*-(1 -Benzylpiperidin-4-yl)propionamid;
Methyl-3-(4-propionamidopiperidin-1-yl)propanoat;
*N*-(1 -Phenethylpiperidin-4-yl)furan-2-carboxamid;
*N*-(1 -Phenethylpiperidin-4-yl)furan-3 -carboxamid;
*N*-(1-(2-(Thiophen-2-yl)ethyl)piperidin-4-yl)propionamid;

8- Brom-*N*-(1-phenethylpiperidin-4-yl)octanamid; und
*N*-(1-Phenethylpiperidin-4-yl)benzamid.

**9.** Die Verbindung zur Verwendung nach Anspruch 1, wobei der klinische Zustand Dyskinesie umfasst, die mit der Parkinson-Krankheit verbunden ist.

**10.** Eine Verbindung mit der Formel (III)

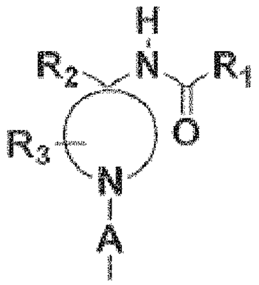

**Ar**, oder (HetAr), oder $COOR_5$ oder $COON(R_6)_2$ (III)

wobei

$R^1$ substituiertes oder unsubstituiertes $C_{1-10}$-Alkyl, -Alkenyl oder -Alkinyl, oder substituiertes oder unsubstituiertes Aryl oder Heteroaryl ist;
$R^2$ H, $-CH_2O-C_{1-4}$-Alkyl; $COO-C_{1-4}$-Alkyl; $-CONR_4$ ist;
$R^3$ H, substituiertes oder unsubstituiertes $C_1$-$C_{10}$-Alkyl, -Alkinyl oder substituiertes oder unsubstituiertes Aryl oder Hetaryl ist;
A substituiertes oder unsubstituiertes $C_{1-10}$-Alkylen, -Alkinylen ist;
Ar oder HetAr ein substituiertes oder unsubstituiertes monocyclisches oder polycyclisches aromatisches oder heteroaromatisches Teil ist;
$COOR_5$ oder $CON(R_6)_2$, wobei $R_5$ und $R_6$ H, substituiertes oder unsubstituiertes $C_1$-$C_{10}$-Alkyl, -Alkinyl, oder substituiertes oder unsubstituiertes Aryl oder Hetaryl sind;
der zentrale stickstoffhaltige Ring ein substituierter oder unsubstituierter 5- bis 7-gliedriger heterocyclischer Ring ist;

die ausgewählt ist aus der Gruppe bestehend aus:

*N*-(1 -Phenethylpiperidin-4-yl)propionamid;
*N*-(1-Phenethylpiperidin-4-yl) propionamidoxalat;
*N*-(1-Benzylpiperidin-4-yl)propionamid;
Methyl-3-(4-propionamidopiperidin-1-yl)propanoat;
*N*-(1-Phenethylpiperidin-4-yl)furan-2-carboxamid;
*N* (1-Phenethylpiperidin-4-yl)furan-3-carboxamid;
*N*-(1-(2-(Thiophen-2-yl)ethyl)piperidin-4-yl)propionamid; und
8-Brom-*N*-(1-phenethylpiperidin-4-yl)octanamid.

**Revendications**

**1.** Un composé ayant la formule :

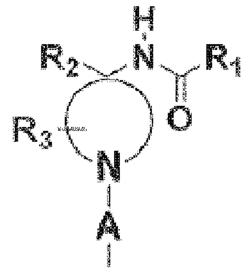

**Ar**, ou (HétAr), ou $COOR_5$ ou $COON(R_6)_2$ (III)

dans laquelle

$R^1$ est un groupe alkyle, alcényle ou alcynyle en $C_{1-10}$ substitué ou non substitué, ou aryle ou hétéroaryle substitué ou non substitué ;
$R^2$ est H, alkyle en $-CH_2O-C_{1-4}$ ; alkyle en $COO-C_{1-4}$ ; $-CONR_4$ ;
$R^3$ est H, alkyle, alcynyle en $C_1$ à $C_{10}$ substitué ou non substitué, aryle ou hétaryle substitué ou non substitué ;
A est alkylène, alcynylène en $C_{1-10}$ substitué ou non substitué ;
Ar ou HétAr est un fragment aromatique ou hétéroaromatique monocyclique ou polycyclique substitué ou non substitué ;
$COOR_5$, ou $CON(R_6)_2$, où $R_5$ et $R_6$ sont H, alkyle, alcynyle en $C_1$ à $C_{10}$, substitué ou non substitué, ou aryle ou hétaryle substitué ou non substitué ;
l'anneau contenant de l'azote central est un anneau hétérocyclique à 5 à 7 chaînons substitué ou non substitué ;
et des sels pharmaceutiquement acceptables dudit composé ;

destiné à être utilisé dans le traitement d'un état clinique associé à l'abus d'opioïdes, un développement de la tolérance et de la dépendance aux opioïdes, une constipation induite par les opioïdes, l'abus de l'alcool, l'abus d'opioïdes, l'abus de la cocaïne, une dépression, une dépression de réponse immunitaire induite par les opioïdes, une dépression respiratoire induite par une surdose d'opioïdes, des symptômes de sevrage de la nicotine, l'obésité, une psychose, une dyskinésie associée à la maladie de Parkinson, la maladie de Raynaud, une sclérodermie ou une combinaison de ceux-ci, par modulation des récepteurs opioïdes $\mu$, $\delta$, $\kappa$ et des adrénorécepteurs $\alpha 2$.

2. Le composé pour l'utilisation selon la revendication 1, dans lequel le composé appartient aux N-(1-aryléthylpipéridine-4-yl)acylamides.

3. Le composé pour l'utilisation selon la revendication 1, dans lequel le composé est le N-(1-phénéthylpipéridin-4-yl)propionamide.

4. Le composé pour l'utilisation selon la revendication 1, dans lequel le composé est l'oxalate de N-(1-phénéthylpipéridin-4-yl)propionamide.

5. Le composé pour l'utilisation selon la revendication 1, qui est un composé de la formule :

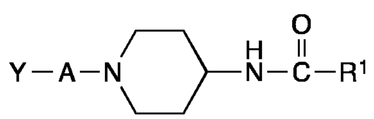

ou un sel pharmaceutiquement acceptable de celui-ci, dans lequel

$R^1$ est un alkyle en $C_{1-10}$ facultativement substitué, un aryle facultativement substitué ou un hétéroaryle facultativement substitué ;
A est un alkylène en $C_{1-10}$ ; et
Y est un aryle facultativement substitué, un hétéroaryle facultativement substitué, ou un fragment de formule $-C(=O)-X^1$, dans laquelle $X^1$ est $-OR^5$ ou $-NR^6R^6$, où chacun parmi $R^5$ et $R^6$ est H ou alkyle en $C_{1-10}$.

6. Le composé pour l'utilisation selon la revendication 5, dans lequel A est l'éthylène.

**7.** Le composé pour l'utilisation selon la revendication 5, dans lequel Y est choisi dans le groupe constitué par le phényle, le thiophén-2-yle et un fragment de formule -C(=O)-OR$^5$, dans laquelle R$^5$ est alkyle en $C_{1-10}$.

**8.** Le composé pour l'utilisation selon la revendication 1, choisi dans le groupe constitué par :

*N*-(1-benzylpipéridin-4-yl)propionamide ;
3-(4-propionamidopipéridin-1-yl)propanoate de méthyle ;
*N* (1-phénéthylpipéridin-4-yl)furan-2-carboxamide ;
*N*-(1-phénéthylpipéridin-4-yl)furan-3-carboxamide ;
*N*-(1-(2-(thiophén-2-yl)éthyl)pipéridin-4-yl)propionamide ;
8-bromo-*N*-(1-phénéthylpipéridin-4-yl)octanamide ; et
*N*-(1-phénéthylpipéridin-4-yl)benzamide.

**9.** Le composé pour l'utilisation selon la revendication 1, dans lequel ledit état clinique comprend une dyskinésie associée à la maladie de Parkinson.

**10.** Un composé ayant la formule (III)

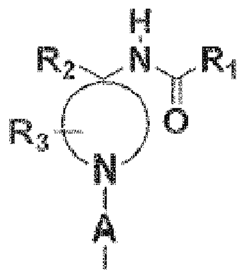

**Ar**, ou (HétAr), ou COOR$_5$ ou COON(R$_6$)$_2$ (III)

dans laquelle

R$^1$ est un alkyle, alcényle ou alcynyle en $C_{1-10}$ substitué ou non substitué, ou un groupe aryle ou hétéroaryle substitué ou non substitué ;
R$^2$ est H, alkyle en -$CH_2O$-$C_{1-4}$ ; alkyle en COO-$C_{1-4}$ ; -CONR$_4$ ;
R$^3$ est H, alkyle, alcynyle en $C_1$ à $C_{10}$ substitué ou non substitué, aryle ou hétaryle substitué ou non substitué ;
A est alkylène, alcynylène en $C_{1-10}$ substitué ou non substitué ;
Ar ou HétAr est un fragment aromatique ou hétéroaromatique monocyclique ou polycyclique substitué ou non substitué ;
COOR$_5$, ou CON(R$_6$)$_2$, où R$_5$ et R$_6$ sont H, alkyle, alcynyle en $C_1$ à $C_{10}$ substitué ou non substitué, ou aryle ou hétaryle substitué ou non substitué ;
l'anneau contenant de l'azote central est un anneau hétérocyclique à 5 à 7 chaînons substitué ou non substitué ;

qui est choisi dans le groupe constitué par :

*N*-(1-phénéthylpipéridin-4-yl)propionamide ;
oxalate de *N*-(1-phénéthylpipéridin-4-yl)propionamide ;
*N*-(1-benzylpipéridin-4-yl)propionamide ;
3-(4-propionamidopipéridin-1-yl)propanoate de méthyle ;
*N* (1-phénéthylpipéridin-4-yl)furan-2-carboxamide ;
*N*-(1-phénéthylpipéridin-4-yl)furan-3-carboxamide ;
*N*-(1-(2-(thiophén-2-yl)éthyl)pipéridin-4-yl)propionamide ; et
8-bromo-*N*-(1-phénéthylpipéridin-4-yl)octanamide.

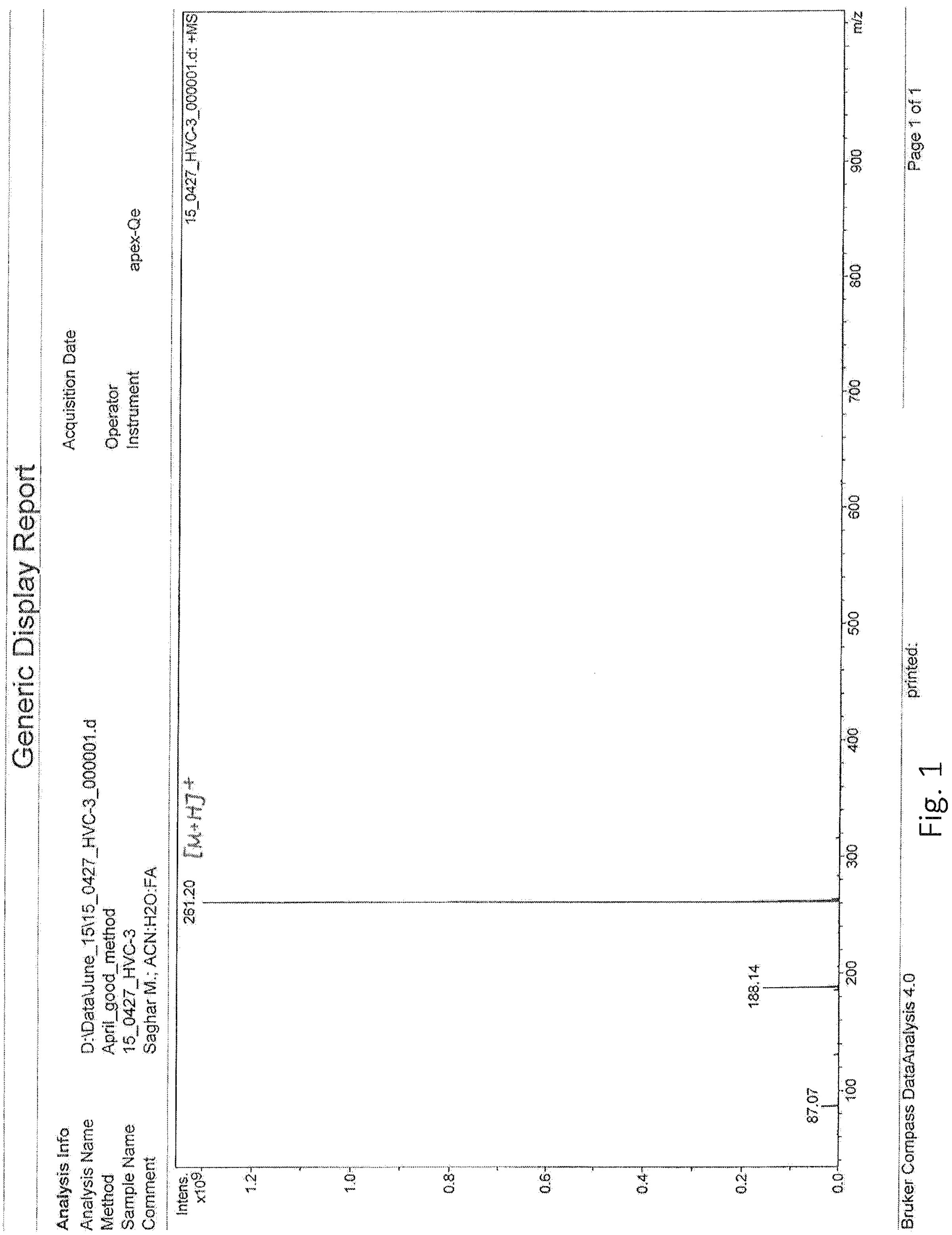
Generic Display Report
Analysis Info
Analysis Name D:\Data\June_15\15_0427_HVC-3_000001.d
Method April_good_method
Sample Name 15_0427_HVC-3
Comment Saghar M., ACN:H2O:FA
Acquisition Date
Operator
Instrument apex-Qe
Intens. x10⁹
1.2
1.0
0.8
0.6
0.4
0.2
0.0
15_0427_HVC-3_000001.d: +MS
87.07
188.14
261.20
[M+H]+
100
200
300
400
500
600
700
800
900
m/z
Bruker Compass DataAnalysis 4.0
printed:
Page 1 of 1

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERGE et al.** *J. Pharm. Sciences,* 1977, vol. 6, 1-19
- **BRUKER.** APEX2. Bruker AXS Inc, 2007
- **BRUKER.** SAINT. Bruker AXS Inc, 2007
- **SHELDRICK, G. M.** SADABS. University of Göttingen, 1996
- **SHELDRICK, G. M.** *Acta Cryst,* 2008, vol. A64, 112-122
- **MACRAE, C. F. ; BRUNO, I. J. ; CHISHOLM, J. A. ; EDGINGTON, P. R. ; MCCABE, P ; PIDCOCK, E. ; RODRIGUEZ-MONGE, L ; TAYLOR, R. ; VAN DE STREEK, J. ; WOOD, P. A.** *J. Appl. Cryst.,* 2008, vol. 41, 466-470
- **SPEK, A. L.** *J. Appl. Cryst.,* 2003, vol. 36, 7-13
- **DOLOMANOV, O.V. ; BOURHIS, L.J. ; GILDEA, R.J. ; HOWARD, J.A.K. ; PUSCHMANN H.** *J. Appl. Cryst.,* 2008, vol. 42, 339-341
- **DOLOMANOV, O.V. ; BOURHIS, L.J. ; GILDEA, R.J ; HOWARD, J.A.K. ; PUSCHMANN, H.** *J. Appl. Cryst.,* 2009, vol. 42, 339-341
- **SHELDRICK, G.M.** *Acta Cryst,* 2008, vol. A64, 112-122
- **WANG, J.B. et al.** *FEBS Lett,* 1994, vol. 338, 217-222
- **AVIDOR-REISS, T. et al.** *FEBS Lett,* 1995, vol. 361, 70-74
- **EASON, M.G. et al.** *J. Biol. Chem.,* 1992, vol. 267, 15795-15801
- **LAW, P.Y. ; LOH, H.H.** *Mol Pharmacol.,* 1993, vol. 43, 684-693
- **UMBUZEIRO, G. et al.** Comparison of the Salmonella/microsome micro-suspension assay with the new microplate fluctuation protocol for testing the mutagenicity of environmental samples. *Environ. Mol. Mutagen.,* 2010, vol. 51 (1), 31-38